# EUROPEAN PATENT SPECIFICATION

(11) **EP 2 046 358 B1**
(45) Date of publication and mention of the grant of the patent: **22.07.2015**
(21) Application number: 07789079.6
(22) Date of filing: 27.07.2007
(51) Int. Cl.: A61K 36/534, A61K 31/513, A61K 31/401, A61K 31/662, A61K 31/196, A61P 25/02, A61P 25/04

(54) **INDUCTION OF ANALGESIA IN NEUROPATHIC PAIN**
AUSLÖSUNG VON ANALGESIE BEI NEUROPATHISCHEN SCHMERZEN
INDUCTION D'UNE ANALGÉSIE DANS UNE DOULEUR NEUROPATHIQUE

(30) Priority: 29.07.2006 GB 0615136
(43) Date of publication of application: 15.04.2009
(73) Proprietor: The University Court Of The University of Edinburgh, South Bridge Edinburgh EH8 9YL (GB)
(72) Inventor: FLEETWOOD-WALKER, Susan, Edinburgh EH9 1QH (GB); MITCHELL, Rory, Edinburgh EH8 9XD (GB); PROUDFOOT, Clare, W., J., Hitchen SGS 1RY (GB)
(74) Representative: Chapman, Paul Gilmour
(86) International application number: PCT/GB2007/002877
(87) International publication number: WO 2008/015403

(56) References cited:
- WO-A-2005/002582
- PROUDFOOT ET AL: "Analgesia Mediated by the TRPM8 Cold Receptor in Chronic Neuropathic Pain" CURRENT BIOLOGY, CURRENT SCIENCE,, GB, vol. 16, no. 16, 22 August 2006 (2006-08-22), pages 1591-1605, XP005606961 ISSN: 0960-9822
- WEI E T ET AL: "AG-3-5: A CHEMICAL PRODUCING SENSATIONS OF COLD" JOURNAL OF PHARMACY AND PHARMACOLOGY, LONDON, GB, vol. 35, no. 2, 1983, pages 110-112, XP009029151 ISSN: 0022-3573
- MACPHERSON ET AL: "More than cool: Promiscuous relationships of menthol and other sensory compounds" MOLECULAR AND CELLULAR NEUROSCIENCES, SAN DIEGO, US, vol. 32, no. 4, 7 July 2006 (2006-07-07), pages 335-343, XP005599220 ISSN: 1044-7431
- ACHER F C ET AL: "Synthesis and pharmacological characterization of aminocyclopentanetricarboxylic acids: new tools to discriminate between metabotropic glutamate receptor subtypes." JOURNAL OF MEDICINAL CHEMISTRY 12 SEP 1997, vol. 40, no. 19, 12 September 1997 (1997-09-12), pages 3119-3129, XP002457201 ISSN: 0022-2623
- DANG K ET AL: "Interaction of group I mGlu and NMDA receptor agonists within the dorsal horn of the spinal cord of the juvenile rat." BRITISH JOURNAL OF PHARMACOLOGY MAY 2002, vol. 136, no. 2, May 2002 (2002-05), pages 248-254, XP002457202 ISSN: 0007-1188
- FISHER KIM ET AL: "Antinociceptive effects following intrathecal pretreatment with selective metabotropic glutamate receptor compounds in a rat model of neuropathic pain." PHARMACOLOGY, BIOCHEMISTRY, AND BEHAVIOR SEP 2002, vol. 73, no. 2, September 2002 (2002-09), pages 411-418, XP002457203 ISSN: 0091-3057 cited in the application
- GALEOTTI NICOLETTA ET AL: "Menthol: a natural analgesic compound." NEUROSCIENCE LETTERS 12 APR 2002, vol. 322, no. 3, 12 April 2002 (2002-04-12), pages 145-148, XP002457204 ISSN: 0304-3940 cited in the application
- VIEIRA ET AL: "Monocytes and plasma tissue factor levels in normal individuals and patients with deep venous thrombosis of the lower limbs: Potential diagnostic tools?", THROMBOSIS RESEARCH, TARRYTOWN, NY, US, vol. 119, no. 2, 21 November 2006 (2006-11-21), pages 157-165, XP005774385, ISSN: 0049-3848
- PEIER ANDREA M ET AL: "A TRP channel that senses cold stimuli and menthol", CELL, CELL PRESS, US, vol. 108, no. 5, 8 March 2002 (2002-03-08) , pages 705-715, XP002246274, ISSN: 0092-8674, DOI: 10.1016/S0092-8674(02)00652-9
- MIZUSHIMA T ET AL: "Noxious cold stimulation induces mitogen-activated protein kinase activation in transient receptor potential (TRP) channels TRPA1- and TRPM8-containing small sensory neurons", NEUROSCIENCE, NEW YORK, NY, US, vol. 140, no. 4, 21 July 2006 (2006-07-21) , pages 1337-1348, XP024986833, ISSN: 0306-4522, DOI: 10.1016/J.NEUROSCIENCE.2006.03.024 [retrieved on 2006-01-01]

## Description

### FIELD OF THE INVENTION

The present invention relates to agents which are capable of inducing analgesia in chronic neuropathic pain, associated methods and uses thereof.

### BACKGROUND

Chronic neuropathic pain arising from peripheral nerve damage is a severe clinical problem with limited treatment options^{[1]}. Changes in both damaged and undamaged primary afferent neurons as well as central (spinal cord) sensitisation lead to hyperalgesia (accentuated responses to painful stimuli), allodynia (pain in response to normally innocuous stimuli) and spontaneous pain.

Since Hippocrates and Galen^{[2,3]}, sporadic reports have described the use of cooling to produce analgesia . Clinical trials show beneficial effects of cooling on chronic back pain, dental pain, post-opera40tive pain, and muscle injuries^{[5]}. Preparations containing menthol, which produces a cool sensation, are used topically to relieve neuralgia in traditional Chinese and European medicine^{[6,7]}. Mint oil has been reported to alleviate thermally-elicited pain and post-herpetic neuralgia^{[8.9]} and oral menthol can cause short-term analgesia^{[10]}. Furthermore, in mice, oral or intracerebroventricular application of menthol decreased nociceptive responses in the hot-plate test and acetic acid writhing test^{[11]}.

The recent isolation of the TRP (transient receptor potential) cation channels present in primary sensory neurons has revolutionised our understanding of cutaneous temperature detection. The best characterised example is the capsaicin- and heat-sensitive TRPV1 receptor^{[12]}, but much less is known about cool-sensitive TRPs^{[13]}. TRPM8 is activated at innocuous cool temperatures (with 50% activation around 18-19°C^{[14]}) and by menthol and icilin^{[15, 16]}. The TRPM8 channel is expressed by a subpopulation of sensory neurons in dorsal root (DRG) and trigeminal ganglia^{[15,16]} where responses to cooling correlate well with mRNA expression and menthol sensitivity^{[17,18,19]}. It seems likely that TRPM8-containing sensory neurons exert their analgesic action at a central spinal site, since cutaneous cooling can prevent pain produced by afferent stimulation^{[4]}. The TRPA1 channel is activated by cooling temperatures beginning 5-6°C lower than that for TRPM8^{[14]} and by noxious chemicals such as cinnamaldehyde and mustard oil^{[20,21,23]}. TRPA1 is also expressed in DRG and trigeminal ganglia^{[14,22]}, but cold sensitivity was found to be unaffected in mice lacking TRPA1^{[23]}, so TRPA1 may be less likely as a candidate for cooling-induced analgesia. Indeed, TRPA1 is more likely a candidate mediator of nociceptive (painful) inputs since another study of TRPA1 knockout mice reported attenuated responses to noxious cold^{[24]} and antisense knockdown studies show a decrease in development of nerve injury- or inflammation-induced hyperalgesia to intense cold stimuli^{[25,26]}.

Glutamate receptor-dependent plasticity in spinal cord neurons commonly underlies chronic pain states with both ionotropic and metabotropic receptors participating at pre- and post-synaptic sites. Although most glutamate receptors are excitatory, the Group II/III metabotropic receptor (mGluR) subtypes exert inhibitory influences, suggesting the hypothesis that they could potentially underpin cooling-induced analgesia. Indeed, Group II and III mGluRs are present in spinal cord largely on afferent terminals, but with some glial and post-synaptic expression^{[27,28]} and their activation inhibits both nerve injury- and inflammation-induced sensitisation of neuronal and behavioural responses^{[29,30,31,32,33]}

The present invention is based on the observation that a marked analgesic effect results from the peripheral or central application of TRPM8 activators in a model of neuropathic pain. Furthermore, while mild cooling and menthol (a TRPM8 activator) have been reported to produce analgesia through unspecified means, it has not previously been established that activation of TRPM8 leads to consistent analgesia in a model of neuropathic pain. TRPM8 levels in DRG and superficial dorsal horn were increased following nerve injury. Analgesia was restricted to injury-sensitised responses and abolished following antisense knockdown of TRPM8. Peripheral application of icilin also activated slowly conducting afferents and suppressed the increased responsiveness of single dorsal horn neurons ipsilateral to nerve injury. In contrast, activation of TRPA 1 produced hyperalgesia (in naive animals and nerve-injured animals). Sensitisation-specific analgesia from TRPM8 activation was also observed in models of pain due to inflammation, afferent demyelination, and activation of TRPV1 or TRPA1 channels in sensory afferents.

### SUMMARY OF THE INVENTION

Transient receptor potential (TRP) cation channels transduce environmental stimuli such as thermal, chemical and mechanical stimuli, into inward currents in sensory neurons that then elicit the appropriate response. TRPV1, TRPV2, TRPV3, TRPV4 and TRPM8 are thermoreceptors providing responses to a range of temperatures both cold and hot. TRPM8 is activated at innocuous cool temperatures (50% activation at around 18-19°C). A number of the TRP channels are also chemosensitive and TRPM8 may be activated upon contact with compounds such as, for example, icilin (1-(2'-hydroxyphenyl)-4-(3"-nitrophenyl)-1,2,3,6-tetrahydropyrimidin-2-one), menthol 1R,2S,5R-(5-methyl-2-[1-methylethyl] cyclohexanol), WS-12 (2-isopropyl-5-methyl-cyclohexanecarboxylic acid (4-methoxyphenyl) amide).

The present inventors have found that activation of the TRPM8 receptor induces analgesia in chronic neuropathic pain. Accordingly, TRPM8 activating agents such as those described herein, may be useful in the treatment of neuropathic pain and/or in the treatment of conditions or diseases which involve neuropathic pain.

Accordingly, and in a first aspect, the present invention provides the use of a transient receptor potential (TRP) M8 cation channel activating agent in the manufacture of a medicament for use in the induction of analgesia in a patient suffering from or experiencing chronic neuropathic pain, wherein the TRPM8 cation channel activating agent is selected from the group consisting of:
(i) icilin (1-(2'-hydroxyphenyl)-4-(3"-nitrophenyl)-1,2,3,6-tetrahydropyrimidin-2-one); and
(ii) menthol 1R,2S,5R-(5-methyl-2-[1-methylethyl] cyclohexanol);
(iii) icilin comprising a modification at the 2' position to include a member selected from the groups consisting of
   (v) C₁-C₄ alkyloxy;
   (vi) NO₂;
   (vii) =O; and
   (viii) NH₂;
(iv) methoxyicilin.
.

In a second aspect, the present invention provides a transient receptor potential (TRP) M8 cation channel activating agent selected from the group consisting of:
(i) icilin (1-(2'-hydroxyphenyl)-4-(3"-nitrophenyl)-1,2,3,6-tetrahydropyrimidin-2-one); and
(ii) menthol 1R,2S,5R-(5-methyl-2-[1-methylethyl] cyclohexanol);
(iii) icilin comprising a modification at the 2' position to include a member selected from the groups consisting of
   (i) C₁-C₄ alkyloxy;
   (ii) NO₂;
   (iii) =O; and
   (iv) NH₂;
(iv) methoxyicilin;
for use in inducing analgesia in a patient suffering from or experiencing chronic neuropathic pain.

It is to be understood that the term "neuropathic pain" refers to pain initiated or caused by a primary lesion or dysfunction in the nervous system or peripheral nervous system (peripheral neuropathic pain). Chronic neuropathic pain therefore refers to a continual or long-lasting neuropathic pain caused by a primary lesion or dysfunction in the nervous system or peripheral nervous system.

Neuropathic pain may result in conditions such as hyperalgesia where there is an accentuated response to painful stimuli and/or allodynia where an innocuous stimulus produces pain.

As such, the present invention may provide a means of treating the chronic neuropathic pain associated with conditions such as, for example, allodynia. This is surprising as the application of cold (which is also known to activate TRPM8) to treat the chronic neuropathic pain associated with allodynia, is known to induce pain. In contrast, the use of the TRPM8 activating agents described herein does not induce pain but alleviates the symptoms of chronic neuropathic pain.

Additionally, or alternatively, neuropathic pain may result in spontaneous pain. Accordingly, the identification and subsequent use of an agent capable of inducing analgesia in subjects suffering from or experiencing chronic neuropathic pain may provide an effective therapy for these conditions.

Chronic neuropathic pain is a phenomenon associated with changes in the central nervous system and as such, the observation that a peripherally applied TRPM8 activating agent is capable of inducing analgesia in patients suffering from or experiencing chronic neuropathic pain, is unexpected.

TRPM8 is activated by, for example, menthol, and in particular (-)-menthol, however other compounds such as 1,2,3,6-tetrahydropyrimidine-2-one compounds also activate TRPM8 receptors. As such, compounds such as these may be capable of inducing analgesia in instances of chronic neuropathic pain. An exemplary TRPM8 activating agent is the 1,2,3,6-tetrahydropyrimidine-2-one compound, icilin (1-(2'-hydroxyphenyl)-4-(3"-nitrophenyl)-1,2,3,6-tetrahydropyrimidin-2-one). The skilled man will understand that the chemical structure of icilin may be modified at, for example, the 2' position, to include a member independently selected from the group consisiting of C₁-C₄ alkyloxy, OH, NO₂, =O and NH₂.

The present invention should be understood to encompass, among others, the icilin derivative methoxy (MeO)-icilin (1-(2'-methoxyphenyl)-4-(3"-nitrophenyl)-1,2,3,6-tetrahydropyrimidin-2-one).

Thus, in one embodiment, there is provided a use of a 1,2,3,6-tetrahydropyrimidine-2-one compound in accordance with claim 2 for the manufacture of a medicament for the induction of analgesia in chronic neuropathic pain, wherein, for example, the 1,2,3,6-tetrahydropyrimidine-2-one compound is icilin or one of the claimed derivatives (for example methoxy-icilin).

In a further embodiment, there is provided a use of a compound selected from the group consisting of:
menthol ((+) or (-)-menthol);
in the manufacture of a medicament for the induction of analgesia in chronic neuropathic pain.

In addition, it should be understood that a further advantage of the compounds described herein is that, repeated dosing does not result in desensitization. In other words, although the analgesic effect of a single application may reduce over time, the response to a further or further application(s) remains intact.

The term "chronic neuropathic pain" may be taken to include a number of specific pain states and a non-exhaustive list of those types of pain which are to be encompassed by this term, is provided below.

### Trauma-induced neuropathic pain.

This particular type of pain may be taken to include pain which arises as a result of physical trauma to peripheral nerves occurring, for example, as a result of injury (accidental or otherwise) or as a result of, for example, surgery where nerves may be subject to cut, crush, constriction, transection or stretch (for example, brachial plexus damage).

### Demyelination-induced pain.

This is a chronic pain state which results from peripheral demyelination of neurones, which may occur as a result of demyelinating diseases such as, for example, Guillain-Barré syndrome and Charcot-Marie-Tooth syndrome^{[34]}.

### Inflammatory pain states.

Pain states may arise from inflammation in soft tissues or joints and may involve damage to the peripheral nerves supplying the bone and/or joint capsule. Conditions such as arthritis may give rise to inflammatory pain states.

### Phantom-limb pain

This pain state may occur in those patients who have undergone an amputation. In such cases, pain, which consists solely or partly of a neuropathic element, is perceived by the patient to emanate from the amputated body part.

### Neuropathic pain linked to cancer

In some cases, growing tumours can constrict or damage nerves leading to neuropathic pain.

### Chemotherapy-induced neuropathy

Certain chemotherapeutic agents, for example those used to treat cancer or HIV, may damage nerves causing neuropathic pain. Such chemotherapeutic agents include, for example, vinca alkaloids^{[35]}.

### Back Pain

Chronic states of back pain may arise, for example, from damage or pressure on peripheral nerves.

### Bone pain and cancer-induced bone pain

Bone cancer pain may arise in humans from either primary bone tumours or more commonly from skeletal metastases from breast, prostate and lung carcinomas. Bone pain is the most common complication of metastatic bone disease. The pain may be caused by structural damage, periosteal irritation, nerve entrapment and nerve damage. Tumours growing within bone injure and destroy the sensory nerve afferents which innervate the bone, resulting in a neuropathic pain state^{[36,37,38]}.

As previously stated, the present invention represents an unexpected finding as, even though chronic neuropathic pain states result from changes which occur at the level of the central nervous system, the application of a TRPM8 activating agent at a peripheral site (i.e. a site distal to the central nervous system, for example the skin (i.e. topically)) may induce an analgesic effect. Furthermore, since the association between TRPM8 activating agents and the induction of analgesia in chronic neuropathic pain has not previously been recognised, in addition to the peripheral application of a TRPM8 activating agent, central, intrathecal or epidural administration of TRPM8 activating agents may also induce analgesia in patients suffering from or experiencing chronic neuropathic pain.

Advantageously therefore, the TRPM8 activating agent may be administered topically, intrathecally (i.e. directly into the spinal cavity) or as an epidural. Accordingly, the TRPM8 activating agents of the present invention may be formulated as sterile pharmaceutical compositions comprising a pharmaceutically acceptable carrier or excipient. Such carriers or excipients are well known to one of skill in the art and may include, for example, water, saline, phosphate buffered saline, dextrose, glycerol, ethanol, ion exchangers, alumina, aluminium stearate, lecithin, serum proteins, such as serum albumin, buffer substances such as phosphates, glycine, sorbic acid, potassium sorbate, partial glyceride mixtures of saturated vegetable fatty acids, water salts or electrolytes, such as protamine sulphate, disodium hydrogen phosphate, potassium hydrogen phosphate, sodium chloride, zinc salts, colloidal silica, magnesium trisilicate, polyvinyl pyrrolidone, cellulose-based substances, polyethylene glycon, sodium carboxymethylcellulose, polyacrylates, waxes, polyethylene-polypropylene-block polymers, polyethylene glycol and wool fat and the like, or combinations thereof.

TRPM8-activating agents may be administered in combination with another treatment. By way of example, as stated above, chronic neuropathic pain can result from the use of chemotherapeutic agents to treat cancer. As such, by combining a chemotherapeutic treatment with a medicament comprising a TRPM8-activating agent, it may be possible to reduce the amount of chronic neuropathic pain experienced by a person being treated for cancer. Such combinations would be beneficial in any situation where the use of a particular compound, substance or drug (or other form of therapy) results in the development of chronic neuropathic pain.

It may be possible to administer the TRPM8-activating agents described above transdermally, via some form of transdermal delivery device. Such devices are advantageous, particularly for the administration of a TRPM8-activating agent capable of inducing analgesia in chronic neuropathic pain, as they may allow a prolonged period of treatment relative to for example, an oral or intravenous medicament.

Examples of transdermal delivery devices may include, for example, a patch, dressing, bandage or plaster adapted to release a compound or substance through the skin of a patient. A person of skill in the art would be familiar with the materials and techniques which may be used to transdermally deliver a compound or substance and exemplary transdermal delivery devices are provided by GB2185187, US3249109, US3598122, US4144317, US4262003 and US4307717.

By way of example, a TRPM8-activating agent may be combined with some form of matrix or substrate, such as a non-aqueous polymeric carrier, to render it suitable for use in a transdermal delivery system. The TRPM8-activating agent/matrix or substrate mixture may be further strengthened by the use of a woven or knit, nonwoven, relatively open mesh fabric, to produce a patch, bandage, plaster or the like which may be releasably attached to a particular region of a patient's body. In this way, while in contact with a patient's skin, the transdermal delivery device releases the compound or substance through the skin.

It is presently described a method of detecting an agent, potentially useful in the manufacture of a medicament for the induction of analgesia in a patient suffering from or experiencing chronic neuropathic pain, said method comprising the step of;
(a) contacting a test agent or agents with a TRPM8 receptor; and
(b) detecting any activation of the TRPM8 receptor so as to identify an agent or agents which activates the TRPM8 receptor.

By way of example, the above described method may be used to identify agents which may be administered topically for the induction of analgesia in a patient suffering from or experiencing chronic neuropathic pain.

A person of skill in the art would be familiar with those techniques required to determine whether or not a test agent has activated a TRPM8 receptor. For example, activation of a TRPM8 receptor allows entry of positively charged ions into TRPM8-expressing cells and may be measured electrophysiologically as inward currents in voltage clamp experiments or by calcium fluorimetry.

The above detailed method may further comprise the step of testing an agent found to activate the TRPM8 receptor for an analgesic effect. For example, to determine whether or not any agent or agents identified by the above method induce analgesia in chronic neuropathic pain, the agent or agents may be tested in a subject (for example a human or animal subject) known to be suffering from or experiencing chronic neuropathic pain and/or in an animal model. By way of example, rodents with chronic constriction injury (CCI) to the sciatic nerve may provide a suitable animal model system in which to assess the analgesic properties of an agent or agents identified by the above detailed method.

The step of testing an agent found to activate the TRPM8 receptor, may comprise the step of administering the test agent topically to a subject known to be suffering from or experiencing chronic neuropathic pain and/or topically to an animal model. The agent may be applied topically to the skin.

It is also herein described a method of detecting agents which are capable of binding the TRPM8 receptor, said method comprising the steps of:
(iii) contacting a test agent or agents with a TRPM8 receptor; and
(iv) detecting any binding which occurs between the test agent and the TRPM8 receptor.

A person of skill in the art would be familiar with those techniques required to determine whether or not a test agent is capable of binding to a TRPM8 receptor. For example, the displacement of a known TRPM8 binding agent may be exploited to detect the binding of a test agent. In such cases, once a test agent has been contacted with a TRPM8 receptor, a known TRPM8 binding agent, for example an antibody or the like, may also be exposed to the TRPM8 receptor. If the known binding agent does not bind to the TRPM8 receptor, that may indicate that the test agent has bound to the TRPM8 receptor i.e. it has displaced the known TRPM8 binding agent. Additionally or alternatively, assays such as band shift assays may assist in detecting whether a test agent is capable of binding to a TRPM8 receptor.

Additionally, the skilled person would understand that it would be desirable to compare any results obtained from the methods described in the third and fourth aspects above, with those from a control system where the TRPM8 receptor is not contacted with the test agent or agents.

The TRPM8 receptor may be expressed on the surface of a cell cultured so as to provide a cell monolayer. Additionally or alternatively, the TRPM8 receptor may be provided by a recombinant system or via a tissue sample or biopsy. Alternatively an animal, for example a rodent, may be used as the source of the TRPM8 receptor.

The TRPM8 receptor may be expressed by a particular cell line such as, for example, the human prostate carcinoma cell line, LNCaP. More specifically, the results obtained from an assay involving the use of a cell line known to express TRPM8 (such as LNCaP) may be compared with the results obtained from a similar assay using a cell line which either expresses relatively less TRPM8 or does not express TRPM8 at all. Furthermore, the methods may further involve the use of a TRPM8 channel blocking agents.

In addition to the above, the present inventors have determined that the analgesic effects of TRPM8 activation may be centrally mediated. For example, and without wishing to be bound by theory, the effects of TRPM8 activation may be dependant upon a receptor which is capable of modulating, for example, a component of the central nervous system. By way of example, TRPM8 activation may result in the release of glutamate and as such, glutamate receptors in the dorsal horn may underlie the induction of analgesia in chronic neuropathic pain. Accordingly, the effects of TRPM8-activation may be dependant upon a particular type of glutamate receptor, namely the Group II/III metabotropic receptor (mGluR).

Agents capable of directly activating Group II/III mGluRs may include Group II/III mGluR agonists such as, for example, 2R, 4R-APDC, ACPT-III and/or AP-4. In addition, agents capable of activating Group II/III mGluRs indirectly, may include agents such as menthol and/or the 1,2,3,6-tetrahydropyrimidine-2-one compounds (e.g. icilin) described above.

### DETAILED DESCRIPTION OF THE INVENTION

The present invention will now be described by way of example and with reference to the figures which show:

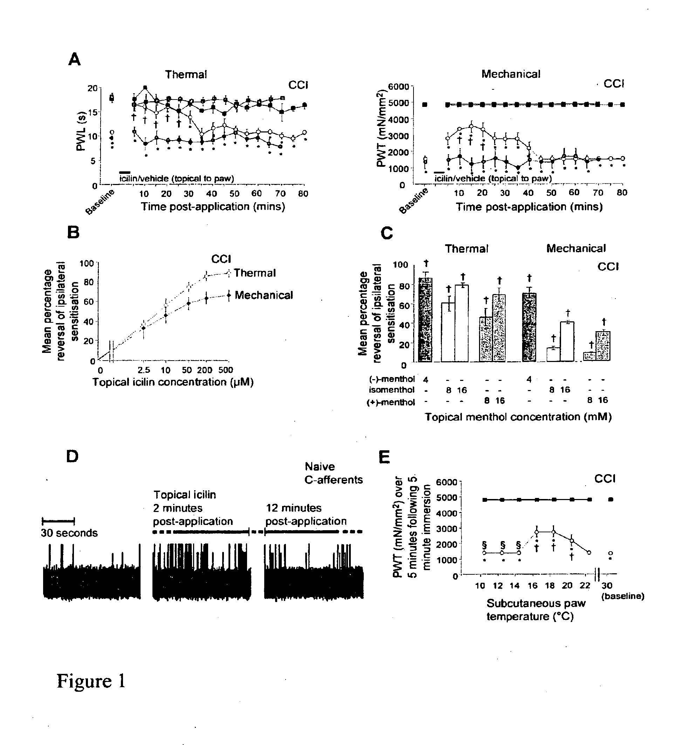

**Figure 1** **Peripheral TRPM8 activation and moderate cooling are analgesic following CCI.**
   A,B,C,E) Behavioural data from CCI animals, shown as mean ± SEM, each graph represents n of 6 animals. A) Paw withdrawal latency (PWL; s) to noxious heat and paw withdrawal threshold (PWT; mN/mm²) to mechanical stimuli before and following 5 min paw immersion in a shallow 30°C water bath containing 80 µM icilin or vehicle; ○: ipsilateral paw plus icilin, 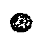 : ipsilateral + vehicle; ■: contralateral plus icilin, 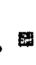 : contralateral plus vehicle, * indicates significant ipsilateral-contralateral differences, † indicates significant difference from pre-drug baseline (p<0.05). B) Concentration-response curve for mean ± SEM percentage reversal of ipsilateral sensitisation for thermal (○) or mechanical (◆) tests calculated over 10-15 min following paw immersion in 2.5-500µM icilin. C) Reversal of ipsilateral sensitisation by paw immersion in (-)-menthol at 4mM, and by higher concentrations of the less potent stereoisomers (+)-menthol and isomenthol (8 and 16mM). Values were calculated over 10-25 min following paw immersion from experiments as shown in A). † indicates significant differences from pre-drug baseline (p<0.05). D) Typical electrophysiological recording from the subpopulation of icilin-responsive C-fibre afferent fibres before, 2 min following topical application of icilin to the receptive field (peak effect) and 12 min later. E) Mechanical analgesia measured over 5 min following 5 min immersion of paws at the indicated temperature range. § denotes spontaneous withdrawal responses during the immersion period, * denotes significant difference from contralateral paw and † from pre-immersion baseline (p<0.05).

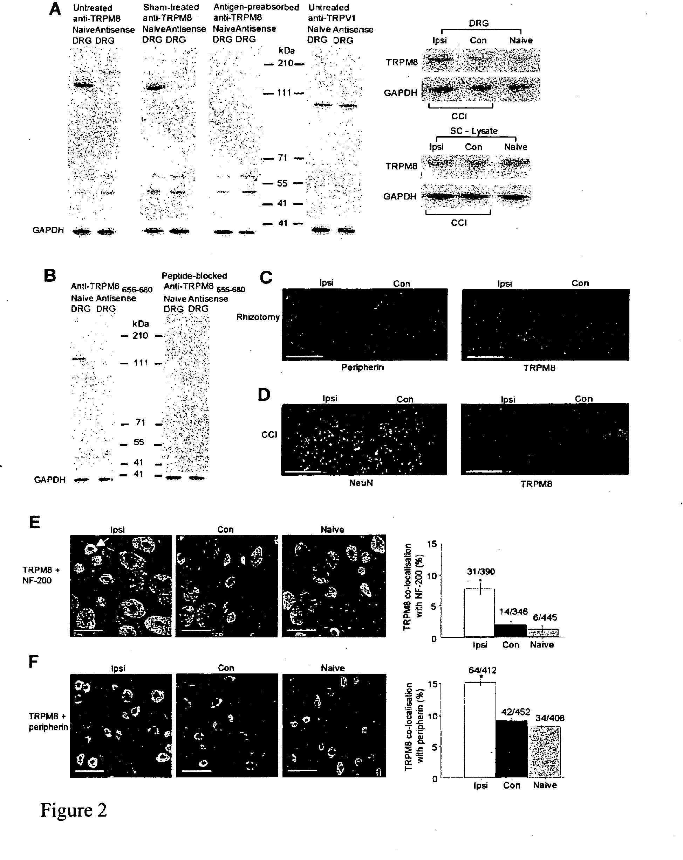

**Figure 2** **TRPM8 immunoreactivity is present in DRG and spinal cord, arises from afferents and is increased ipsilateral to CCI.**
   **(v)** Immunoblots of DRG show whole gels with TRPM8 protein running at 128kDa and additional faint bands at approximately 170, 60 and 50 kDa, in normal rat DRG with specific knockdown of the 128 kDa band in DRG from antisense-treated animals. In additional controls, when the TRPM8 antibody was pre-incubated with membranes from TRPM8-expressing cells, the 128kDa immunoreactive band was removed, whereas sham-treatment had no effect. Blots additionally show GAPDH loading controls. TRPV1 expression (single band at ~90 kDa) was unaltered in DRGs from TRPM8 antisense-treated animals. Immunoblots for TRPM8 protein showed a clear increase in expression of the specific 128kDa band in DRG ipsilateral ('ipsi') to nerve injury compared to contralateral ('con') and I DRG, with no change in GAPDH. Spinal cord (SC) whole lysates showed no discernable changes in TRPM8 levels, however, increased levels ipsilateral to nerve injury were seen in crude particulate fractions. B) Shows Western blots of DRGs from naïve or TRPM8 antisense-treated rats probed with the TRPM8 antibody used for immunohistochemistry in C)-F) below. Pretreatment of the antibody with the antigenic peptide or TRPM8 antisense treatment removed the single specific band at -̂128 kDa. C) L5 spinal cord sections taken 8 days after dorsal rhizotomy were immunostained for peripherin (green) and TRPM8 (red) and showed marked reduction of both proteins ipsilateral to rhizotomy. D) Immunostaining for TRPM8 (red) and the neuronal marker NeuN (green) in spinal dorsal horn from CCI animals showed that TRPM8 was increased ipsilateral to CCI with no change in distribution, whereas NeuN levels were unchanged. Scale bar for C) and D) is 500µm. E,F) Immunohistochemical colocalisation in DRG sections ipsilateral or contralateral to nerve injury and in I animals of TRPM8 (red) with (E) NF-200 (green) or (F) peripherin (green). In naive animals, TRPM8 is mainly located in peripherin-positive C-fibres with little or no apparent expression in myelinated (NF-200) cells. Ipsilateral to nerve injury, TRPM8 expression was increased markedly in small NF-200-positive cells, whereas a lesser increase in TRPM8:peripherin co-expression was also observed. Scale bar 50µm. The bar charts in E) and F) show the percentage co-expression (mean ± SEM) for TRPM8:NF-200 and TRPM8:peripherin respectively; actual cell counts shown above columns. Statistically significant increases in the percentage co-expression values were seen in both cases ipsilateral to CCI, p<0.05 (*).

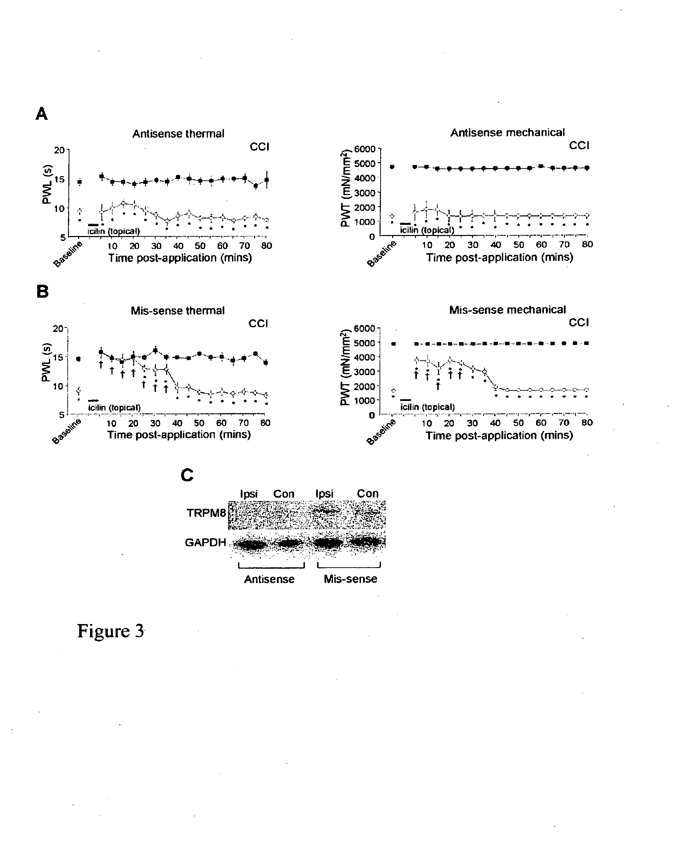

**Figure 3** **Specific TRPM8 knockdown by antisense oligonucleotide prevents icilin-induced analgesia following CCI.**
   A, B) Paw withdrawal latency (PWL, s) to noxious heat and paw withdrawal threshold (PWT; mN/mm²) to mechanical stimuli are shown ipsilateral (○) or contralateral (■) to CCI animals with antisense (n=12) or mis-sense (n=10) treatments. Data show mean ± SEM. A) The analgesia normally elicited by topical icilin was not observed following antisense knockdown of TRPM8 receptor indicated by the persistence of significant ipsilateral-contralateral differences in PWL and PWT (* p<0.05). B) In contrast, following mis-sense treatment, icilin produced a significant reversal of CCI-induced ipsilateral sensitisation in PWL and PWT responses in comparison to baseline values († p<0.05), as in untreated CCI animals (Fig 1A). C) Immunoblots of DRG tissue probed for TRPM8 and GAPDH protein levels following TRPM8 antisense or mis-sense treatment. TRPM8 expression (and the increase in expression normally seen ipsilateral to CCI, Fig 2A) was selectively reduced by antisense, but not mis-sense infusion, whereas GAPDH levels were unchanged.

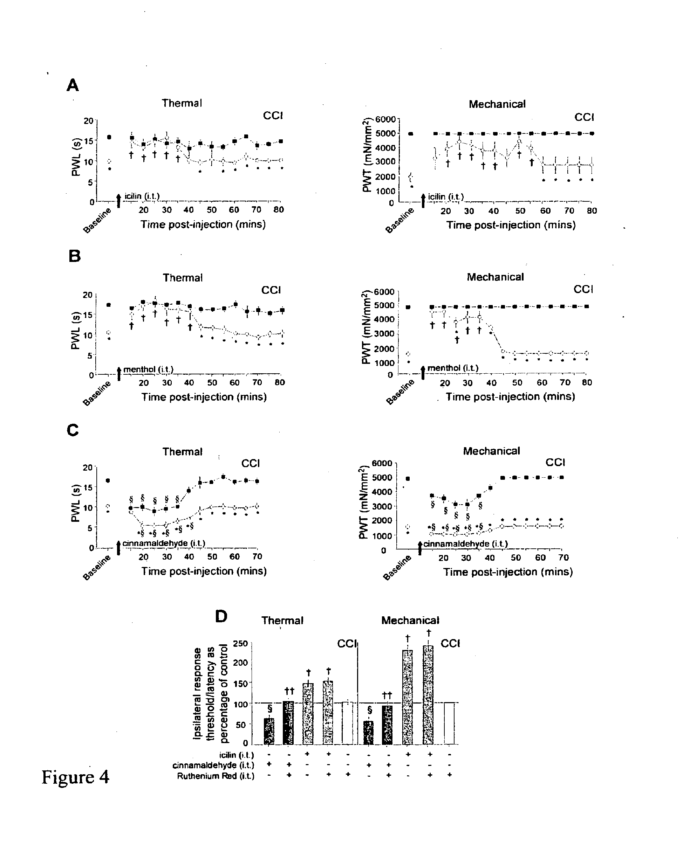

**Figure 4** **Central TRPM8 activation is analgesic following CCI, whereas TRPA1 activation is hyperalgesic.**
   Paw withdrawal latency (PWL, s) to noxious heat and paw withdrawal threshold (PWT, mN/mm²) to mechanical stimuli are shown ipsilateral (○) or contralateral (■) to CCI * denotes significant ipsilateral-contralateral differences (* p<0.05). Data show mean ± SEM and each test represents n of 6 animals unless otherwise indicated. Rats were intrathecally injected at arrow. The TRPM8 activators, A) Icilin (10nmol), and B) (-)-menthol (200nmol) both significantly reversed ipsilateral thermal and mechanical sensitisation in comparison to pre-injection values († p<0.05). C) In contrast, intrathecal application of the TRPA1 activator, cinnamaldehyde (75nmol) produced bilateral hyperalgesia and allodynia in CCI animals. §(p<0.05) shows statistically significant increases in thermal and mechanical reflex responsiveness of both ipsilateral and contralateral paws. D) Shows that Ruthenium Red (0.25 nmol) inhibits cinnamaldehyde (75 nmol)-induced hypersensitivity, but not icilin (10 nmol)-mediated analgesia, ipsilateral to CCI. Values are means ± SEM, n=4. Statistically significant changes in PWL/PWT values due to cinnamaldehyde or icilin compared to pre-drug baseline are shown as (§) and (†) respectively (p<0.05) and for Ruthenium Red-reversal of the effect of cinnamaldehyde (††, p<0.05).

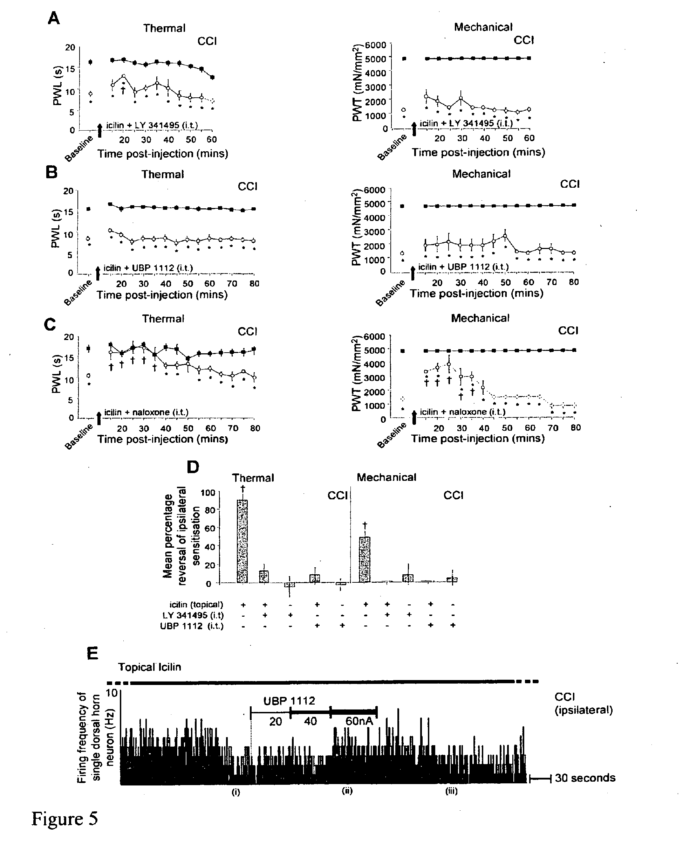

**Figure 5** **Icilin-induced analgesia following CCI is prevented by Group II and III mGluR antagonists.**
   (A-D) Paw withdrawal latency (PWL, s) to noxious heat and paw withdrawal threshold (PWT, mN/mm²) to mechanical stimuli ipsilateral (○) or contralateral (■) to CCI. Data represent mean ± SEM, with an n of 6 animals in each case. Rats were intrathecally injected (at arrow). Icilin (10nmol) was co-administered with A) the group II mGluR antagonist, LY 341495 (5nmol), B) the group III mGluR antagonist, UBP 1112 (10nmol) or C) the opioid receptor antagonist, naloxone (25nmol). A, B) When either LY 341495 or UBP 1112 were co-administered with icilin, they abolished the analgesic effect of icilin as indicated by the persistence of sensitised (* p<0.05) PWT/PWL responses ipsilateral to nerve injury. C) naloxone coadministration with icilin did not prevent the icilin-induced analgesia seen in PWT and PWL responses ipsilateral to nerve injury († p<0.05). * indicates significant ipsilateral-contralateral differences, † indicates significant ipsilateral analgesic effect of drug (p<0.05). D) The analgesic effects of topically applied icilin (200µM at 30 °C) were also reversed by intrathecal injection of Group II/III mGluR antagonists. The figure shows mean % reversal of the ipsilateral/contralateral difference in either PWL or PWT measured over 15-30 min following 5 min topical application of icilin, with or without concurrent intrathecal injection of LY 341495 (5nmol) or UBP 1112 (10nmol), or following mGluR antagonists alone. E) Typical extracellular recording of a single dorsal horn neuron ipsilateral to CCI, responding to continuous motorised brushing of the cutaneous receptive field on the hindpaw and the effects of icilin (200µM at 30 °C) topically applied to an adjacent area of the receptive field. Similar results were observed in 8 out of 12 neurons with examples in both laminae III/IV and lamina I. Neuronal firing is displayed as action potentials per second (Hz) plotted against time. (i): brush-evoked firing in neurons ipsilateral to nerve injury was consistently inhibited by topically applied icilin, (ii): this effect was reversed by ionophoresis of UBP 1112 at 20-60nA, (iii): recovery was observed following removal of the UBP 1112 ejection current. Neurons contralateral to nerve injury were unaffected by icilin and topical vehicle had no effect. In addition, ionophoresis of UBP 1112 alone or saline current controls showed no discernable effect.

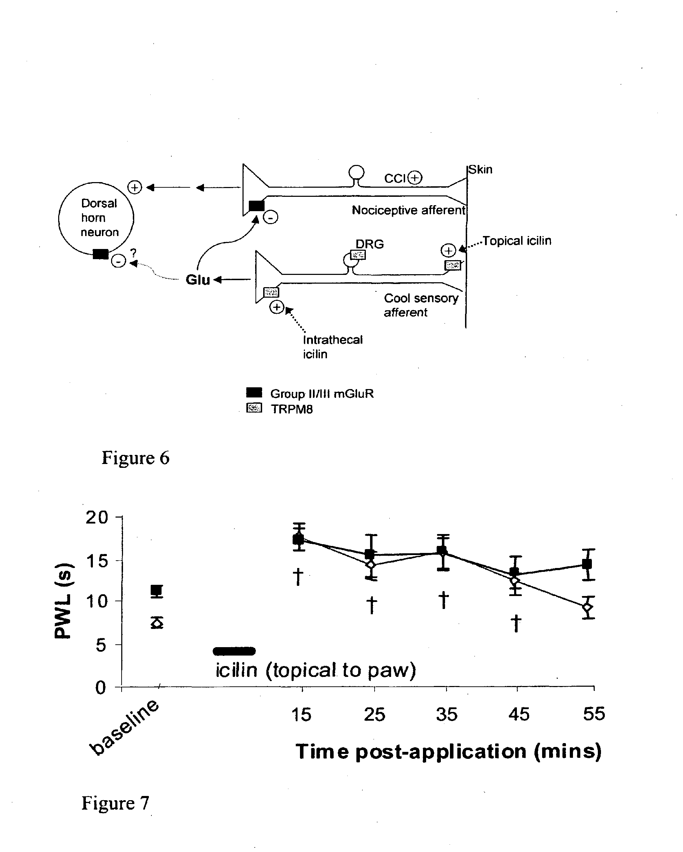

Figure 6 Schematic representation of a possible mechanistic basis for TRPM8-mediated-analgesia following CCI.
   In this simplified hypothetical model, activation of TRPM8 in a subpopulation of afferents, by icilin, menthol or moderate cooling leads to central synaptic release of glutamate which then acts through inhibitory Group II/III mGluR receptors located either pre-synaptically on injury-activated nociceptive afferents or perhaps also post-synaptically on dorsal horn neurons, thereby attenuating neuropathic sensitisation.
**Figure 7****: Topical application of the TRPM8 activator, icilin, causes analgesia in a further neuropathic pain model: the SNL, spinal nerve ligation model.** The SNL model of neuropathic pain was set up according to Kim and Chung 1992 (Pain 50: 355-363). The Hargreaves' test for thermal hyperalgesia was carried out as described above and icilin (200 µM) was applied topically to the ipsilateral (◊) and contralateral (■) paw. Values are shown as means ± SEM. † Indicates statistically significant reversal of sensitisation ipsilateral to nerve injury (see Fig 1). No significant drug effects were seen on reflex withdrawal responses of the contralateral limb.

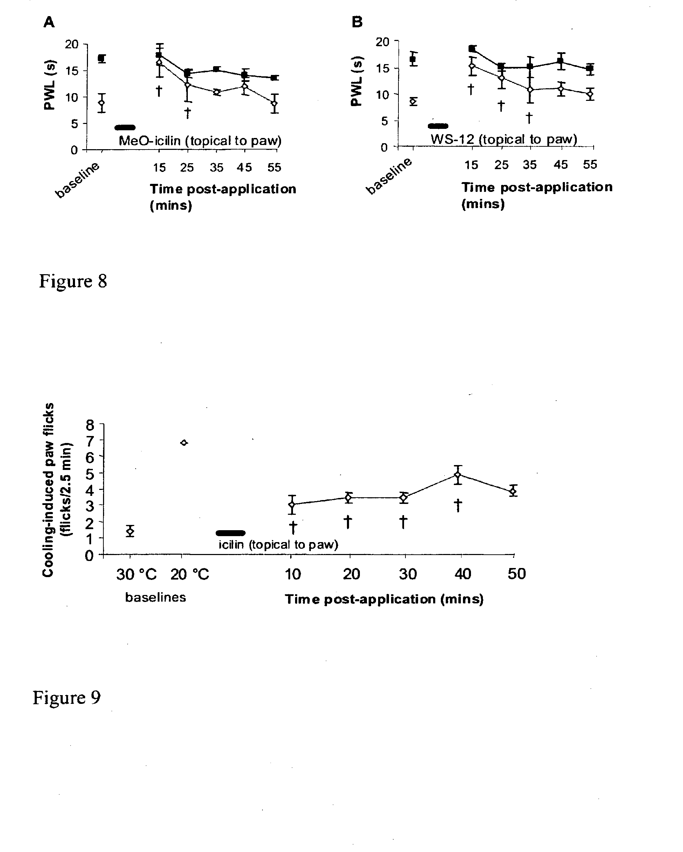

**Figure 8****: Topical application of additional TRPM8 activators, methoxy-icilin and WS-12, also causes analgesia in the CCI model of neuropathic pain.**
   In the CCI model of neuropathic pain, thermal hyperalgesia was assessed by the Hargreaves' test and TRPM8 activators (at a concentration of 30 µM) were applied topically (as in Fig 1). Values are shown as means ± SEM, ipsilateral (◊) or contralateral (■) to nerve injury. † Indicates statistically significant reversal of sensitisation ipsilateral to nerve injury.
**Figure 9****: Topical application of additional TRPM8 activator, icilin, provides analgesia against the cold allodynia elicited in the CCI model of neuropathic pain.**
   In the CCI model of neuropathic pain, cold allodynia was assessed by scoring the number of paw flick-associated paw withdrawals from a Peltier controlled footplate that was reduced in temperature from control (30 °C) to mild cooling (20 °C). Icilin (200 µM) was applied topically (as in Fig 1). Values are shown as means ± SEM, ipsilateral (◊) to nerve injury. † Indicates statistically significant reversal of cool allodynia. Footplate maintenance at 30 °C did not cause any change in baseline levels of flicking paw withdrawals and this was unaffected by topical application of icilin.

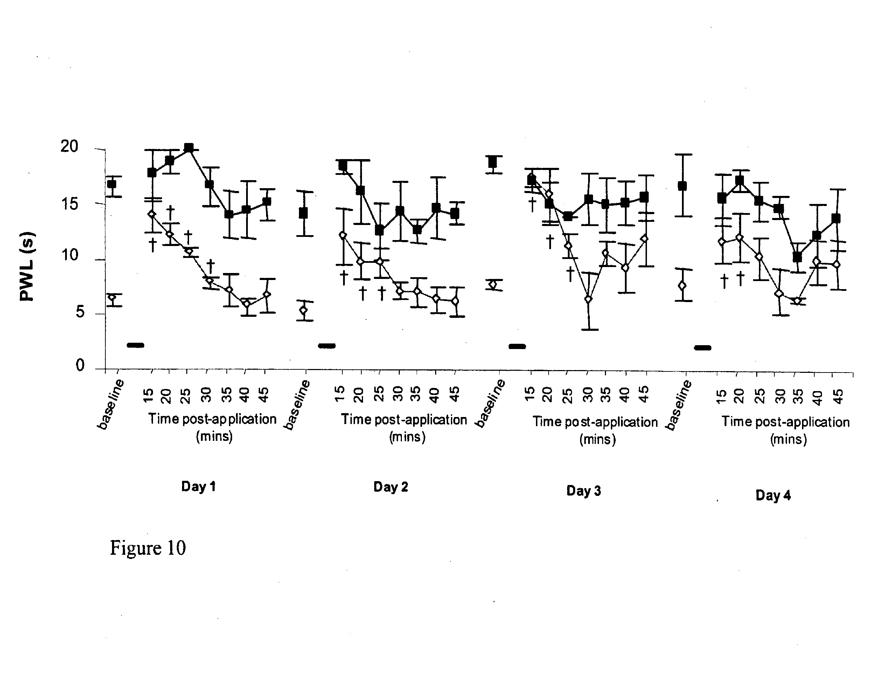

**Figure 10****: Daily repeated topical application of the TRPM8 activator, icilin, consistently causes analgesia in the CCI model of neuropathic pain, demonstrating lack of habituation.**
   In the CCI model of neuropathic pain, thermal hyperalgesia was assessed by the Hargreaves' test and icilin (200 µM) was applied topically (as in Fig 1 over four successive days. Values are shown as means ± SEM, ipsilateral (◊) or contralateral (■) to nerve injury. † Indicates statistically significant reversal of sensitisation ipsilateral to nerve injury.

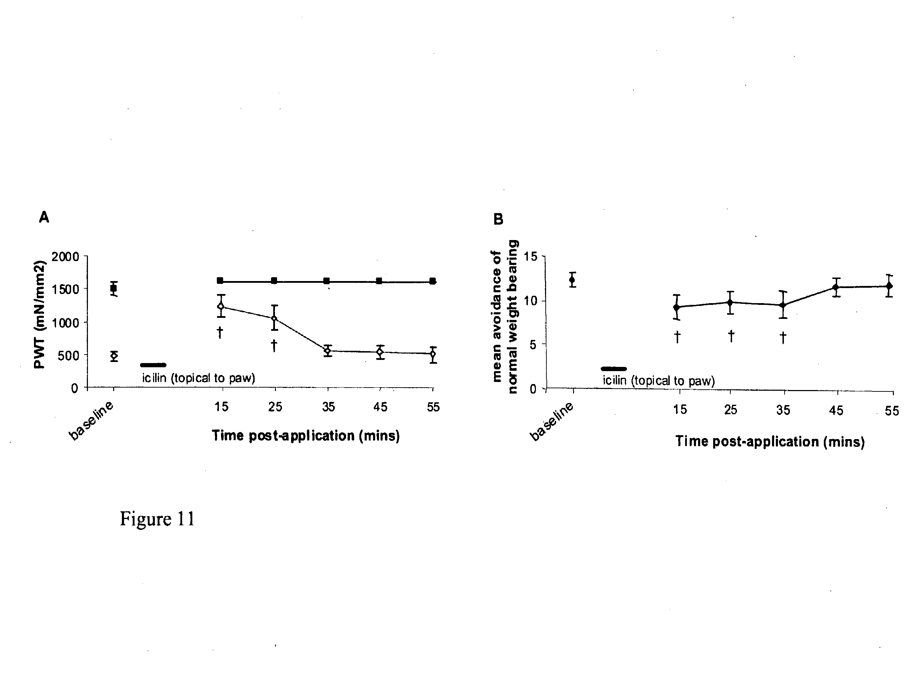

**Figure 11****: Topical application of the TRPM8 activator, icilin, causes analgesia in a model of cancer-induced bone pain.**
   The model of cancer-induced bone pain was set up according to Medhurst et al., 2002 Pain 96: 129-40. In A) von Frey filament testing for mechanical allodynia was carried out as described above (Fig 1) and in B) movement-evoked pain in the injured limb was assessed by scoring the frequency of abnormally prolonged paw lifts in a slow-rotating rotarod test. † Indicates statistically significant reversal of sensitisation ipsilateral to nerve injury.

### Materials & Methods

### Animals

All experiments were conducted in accordance with the UK Animals (Scientific Procedures) Act, 1986, and guidelines of the University of Edinburgh. Adult male Wistar rats (Harlan, UK) weighing between 120-250g were used for all experiments.

### Neuropathic and inflammatory pain models

Pain models were generated under halothane anaesthesia (Zeneca, Cheshire, UK). For the chronic constriction injury (CCI) model of neuropathic pain, four ligatures were tied loosely to constrict the sciatic nerve at mid-thigh level (as described previously^{[39]}). An inflammatory pain model was generated by injecting 100µl of Complete Freund's Adjuvant (CFA, Sigma-Aldrich Ltd) into the ventral surface of the right hindpaw^{[39]}. A model of peripheral demyelination-induced pain was produced by focal application of lysolecithin to the sciatic nerve^{[40]}. Peak behavioural sensitisation was observed post-surgically between days 10-16 for CCI, 1-3 for CFA, and 7-14 for lysolecithin, when pharmacological and electrophysiological experiments and tissue removal were conducted.

### Behavioural testing

Thermal sensitivity was assessed by measuring paw withdrawal latency (PWL, s) in response to a noxious thermal stimulus (Hargreaves' thermal stimulator, Linton Instrumentation, Diss, UK) directed to the hindpaw mid-plantar glabrous surface. Mechanical sensitivity was recorded as the paw withdrawal threshold (PWT, mN/mm²) to calibrated von Frey filaments (Stoelting, Illinois, US), as previously described^{[39]}. Sensitivity to noxious cold was assessed by placing animals in a waterbath with an aluminium floor containing 1cm deep 4°C water and counting the time the paw was held suspended over a 20 s period.

### Intrathecal application of drugs

The following drugs were applied intrathecally in a 50µl volume of saline-based vehicle at 37°C: icilin (2.5-200 µM in saline with 0.2% dimethylformamide, DMF), LY 341495 (100 µM in saline), UBP 1112 (200 µM in saline), 2R, 4R-APDC ((2R, 4R)-4-aminopyrrolidine-2,4-dicarboxylate), 300 µM in saline), ACPT-III ((1R,3R,4S)-1-aminocyclopentane-1,3,4-tricarboxylic acid), 3 mM in saline), AP-4, ((L-(1)-2-amino-4-phosphonobutyric acid), 3 mM in saline), naloxone (0.5 mM in saline), NMDA (75 µM in saline) and ACPC (1-aminocyclopropanecarboxylic acid, 15 µM in saline) (Tocris Cookson, Bristol, UK), (-)-menthol (1R, 2S, 5R-(-)-menthol, 4 mM in saline), cinnamaldehyde (1.5 mM in saline) and Ruthenium Red (5 µM in saline) (Sigma-Aldrich Co. Ltd, UK) and allicin (0.5 mM in saline with 0.5% DMF) and diallyl disulphide (DADS, 1 mM in saline with 0.5% DMF) (LKT Laboratories Inc., St Paul, MN, USA). Drugs were injected into the L5/6 intrathecal space under brief halothane anaesthesia, using a 25-gauge needle microsyringe (BD Biosciences, Oxford, UK), as described previously^{[39]} in animals that were at peak levels of behavioural sensitisation. Behavioural reflex testing commenced 15 min following injection to allow recovery from anaesthesia^{[40,41,42]} and continued every 5 min thereafter until readings returned to baseline levels (n = 6 in each case). We, and others^{[40,41,42]} find complete recovery from anaesthetic by 15 minutes. All appropriate controls were carried out to eliminate the possibility of effects due to vehicle or to injection procedure.

The TRPM8 channel activators, icilin and (-)-menthol were tested in CCI and naïve animals. The effects of icilin were additionally assessed in animals with CFA-induced inflammation or with lysolecithin-induced demyelination. Icilin was also co-administered with the µ-opioid receptor antagonist naloxone, the Group II metabotropic glutamate receptor (mGluR) antagonist, LY 341495, or the Group III mGluR antagonist, UBP 1112. The effects of these antagonists alone as well as effects of the Group II mGluR agonist, 2R, 4R-APDC and the Group III mGluR agonists, ACPT-III and AP-4, were assessed in CCI animals. The TRPA1 channel activator, cinnamaldehyde, alone and with icilin, was tested in CCI animals and in naive animals. Further TRPA1 channel activators, allicin and diallyl disulphide were assessed in naïve animals. Both icilin and cinnamaldehyde effects in CCI animals were also investigated in the additional presence of Ruthenium Red.

### Topical application of drugs

Icilin was applied at concentrations of 2.5-500 µM (in water with 0.2% DMF), by placing CCI or naïve rats unrestrained for 5 min in a 1 cm deep waterbath (sufficient to cover paws), which was thermostatically controlled to a temperature of 30°C, or by very lightly anaesthetising rats and immersing hindpaws in small tubes containing 5 ml icilin (500 µM-5mM, with a vehicle of 45% dimethylformamide in 0.2% aqueous Tween 80) for 5 min, followed by sensory testing for 60-80 mins. The effects of (-)-menthol and its stereoisomers isomenthol (IS, 2R, 5R-menthol,) and (+)-menthol (1S, 2R, 5S (+)-menthol) (4 - 16mM in 80% ethanol) were also assessed. Relevant vehicles were always assessed in similar experiments. As a contrast, the effect of cinnamaldehyde (1.5mM in water) and the effect of cinnamaldehyde with additional icilin (80 µM in water with 0.2% DMF) were assessed in naïve animals. The effects of icilin (80 µM) were further assessed in CCI animals that had undergone antisense and mis-sense treatment for knockdown of TRPM8 or immediately following intrathecal injection of either LY 341495 or UBP 1112. Actual skin temperatures were measured by subcutaneous thermistor probe and were found to equilibrate to around 0.5°C above bath temperature. The effects of brief paw immersion at different temperatures (10-22°C for 5 min) on CCI rats were assessed by mechanical testing. The effects of intrathecal LY 341495 or UBP 1112 on the reversal of ipsilateral sensitisation in CCI following a 16°C cool challenge for 5 min were measured. Reflex testing commenced 5 minutes following challenge, unless animals had been anaesthetised, in which case 15 minutes was allowed for recovery. Six replicate animals were tested in all pharmacological experiments.

### Dorsal rhizotomy

To establish whether TRPM8 expression in spinal cord was predominantly pre- or post-synaptic, a unilateral L2-6 dorsal rhizotomy was performed under anaesthesia, following laminectomy to expose the dorsal roots. Eight days later, tissue was removed and processed for immunohistochemistry.

### Western blots

Experiments were performed by standard procedures as previously described^{[39]}, for more detail see Supplementary material.

### Immunohistochemistry

Experiments were performed as previously described^{[40]}, for more detail see Supplementary material.

### Antisense knockdown of TRPM8

Antisense and mis-sense oligonucleotides were 22-mers with phosphorothioate bonds at the last two positions at 5' and 3' ends (MWG Biotech, Ebersberg, Germany). Antisense extended from base -10 to base +12 relative to the start of the open reading frame for the rat TRPM8 gene: 5' C*T*CGAAGGACATCTTGCCGT*G*G 3', where * represents phosphorothioate linkages. Mis-sense was designed with 4 inversions of C/G or A/T as appropriate at residues 3, 11, 14 and 22, preserving overall G/C content. BLAST searches of both oligonucleotides indicated no significant complementarity to any known gene sequence. 14-day or 7-day osmotic minipumps (for CCI experiments or naïve electrophysiology experiments respectively - Alzet Minipump, models 2002, 2001; Charles River, UK) containing oligonucleotides (1µg/µl in sterile saline) were connected to canulae inserted under the dura of the spinal cord to level L5/6 and produced a predicted infusion rate of 0.5 µl/h. CCI surgery was performed at the same time as minipump implantation. Sensory tests were carried out to assess the time-course of behavioural sensitisation in animals which had also undergone a CCI injury. Icilin was applied topically rather than intrathecally so as to prevent any interference with the infusion canula. Peripheral nerve recordings and tissue harvesting were carried out after an interval of 4-5 days to allow time for protein knockdown.

### Electrophysiology

**Peripheral:** Recordings of saphenous (sensory) nerve were made in naïve animals (n=7) to assess the effects of topical icilin on primary afferents. In addition, recordings were carried out on animals which had undergone TRPM8 antisense or mis-sense treatment beginning 4-5 days previously. Rats were anaesthetised (with 0.6ml 25% urethane /100g, i.p.) and the saphenous nerve was exposed and dissected from its associated vein and artery. Further dissection under liquid paraffin enabled identification of afferent preparations comprising a small number of fibres. The conduction velocity of single identified afferent fibres was determined using bipolar electrodes and the peripheral stimulus technique^{[43]}. Following isolation of preparations, icilin (200µM in water with 0.2% DMF), (-)-menthol (4mM in 25% ethanol) resiniferatoxin (1mM in ethanol) or vehicle alone were applied to the hindlimb receptive fields and neuronal responses recorded using the Chart programme (version 3.6).

**Central:** Recordings of spinal dorsal horn neurons were made in CCI animals, as described previously^{[39]}. Following halothane induction, the jugular vein and trachea were canulated and intravenous anaesthetic delivered: α-chloralose (0.6 mg/kg, Fisher) and urethane (1.2mg/kg, Sigma), with small supplementary doses of α-chloralose as required throughout the experiment. Core body temperature was maintained at 37-38°C by means of a thermostatically controlled heated blanket. The animal was placed in a stereotaxic frame, and the thoracolumbar spinal column was supported using three pairs of swan-necked clamps. A laminectomy was performed at L2-L5, and agar (2% in saline at 37°C) was delivered over exposed cord to increase mechanical stability. Above the recording region the agar and spinal cord dura were removed and liquid paraffin poured into the pool. Extracellular recordings were made from single multireceptive neurons in laminae I-IV through the centre barrel of a seven-barrelled glass microelectrode filled with 4M NaCl (tip-diameter 4-5 mm, DC resistance 5-8 MΩ). The receptive fields of hair-follicle innervated neurons on the distal hindlimb were identified by an innocuous brush stimulus^{[39]}. Icilin (200 µM in water with 0.2% DMF) was applied peripherally to the receptive field of individual recorded neurons, and the effect on neuronal response to a rotating brush was recorded and analysed using Spike2 programme (Version 3.2, CED). The Group III mGluR antagonist UBP 1112 (20 mM in water), pH 8.5, and control 1M NaCl, pH 8.5 were ionophoresed from the side barrels of the electrode using currents of between 20nA and 80nA (Neurophore BH2 ionophoresis system, Medical Systems, Great Neck, NY) to measure effects on neuronal response to icilin.

### Statistics

All data were analysed for statistical significance using Sigmastat software (version 2.03) with p values <0.05 being considered significant. Differences in thermal sensitivity between the paw ipsilateral to nerve injury and the contralateral paw were assessed using Student's t-test. Any effect of drug treatment was analysed by One-way Repeated Measures ANOVA followed by Dunnett's post-hoc multiple comparisons test. The equivalent non-parametric tests for mechanical sensitivity were Wilcoxon rank test for ipsilateral:contralateral differences, and Friedman Repeated Measures ANOVA followed by Dunn's test for changes from pre-drug control values. Western blot densitometric values were compared using the Wilcoxon test, immunohistochemistry cell counts were analysed by One-Way ANOVA, and electrophysiological spike frequencies were analysed by One-Way ANOVA on ranks.

### Western blots

Ipsilateral or contralateral L4-L6 dorsal root ganglia (DRG) were taken from CCI animals (n=6), and from I animals. DRG were also taken from naive and CCI animals that had undergone either antisense or mis-sense treatment to knockdown TRPM8 expression (n=5). Spinal cord from CCI animals was hemisected and samples from naive animals were also processed. Individual samples were weighed and homogenised immediately upon removal. For whole lysate preparations, tissue was homogenised in 20 volumes of Laemmli lysis buffer (Tris (tris-hydroxymethylaminoethane, 50 mM, pH 7.4 with 5% mercaptoethanol and 2% sodium dodecyl sulphate (SDS)), boiled for 5 min and frozen. In some cases with spinal cord samples, a crude particulate fraction was prepared to obtain relative enrichment of membrane-bound compared to cytosolic proteins. Tissue was homogenised in 20 volumes of ice-cold Tris buffer (50 mM, pH 7.4, containing 1% protease inhibitor cocktail III (Calbiochem, Merck Biosciences Ltd., Nottingham, UK) and then centrifuged at 11000g for 45 min at 4°C, before solubilising the pellet in Laemmli buffer. Western blotting was carried out as described previously^{[39]}. Proteins were separated by SDS-PAGE on 3-8% Tris-acetate gradient gels using the (NuPage System, Invitrogen Ltd., Paisley, UK). Blots were incubated with rabbit polyclonal antibodies to TRPM8 (either an antibody raised against TRPM8 residues 278-292 and 1090-1104 (human); 1:500; Abcam Ltd., Cambridge, UK or occasionally an antibody raised against TRPM8 residues 656-680 (rat); 1:100; Phoenix Peptides, Belmont, CA, USA), a rabbit polyclonal antibody to TRPV1 (1:250, Chemicon International Ltd, Harrow, UK) or a mouse monoclonal antibody to glyceraldehyde-3-phosphate dehydrogenase (GAPDH, 1:750; Chemicon) and immunoreactive bands were detected by □eurons□ra-linked secondary antibody and enhanced chemiluminescence. Quantitative densitometry analysis of protein bands was performed using the ScanAnalysis (Elsevier) programme.

Antigen preabsorbtion controls were carried out to assess the specificity of the TRPM8 antibodies, by preincubating the antibody with either membrane preparations from cells heterologously expressing TRPM8 (or control) or the antigenic peptide (or control). COS7 cells were transfected with human TRPM8 in pCMV6-XL4 expression plasmid or empty vector (OriGene Technologies Inc., Rockville, MD, USA) using GeneJuice Reagent (Novagen, Merck Biosciences Ltd., Nottingham, UK) and used 48 h later. Cells were scraped into ice-cold phosphate-buffered saline (PBS) containing 1% protease inhibitor cocktail III (Calbiochem) and homogenised (Ystral homogeniser) before centrifuging at 1000g for 10 min (and discarding the low speed pellet). The supernatant was then centrifuged at 48,000g for 30 min and the pellet from this was resuspended in primary antibody buffer (2% BSA, 0.1% Tween-20 in PBS). Anti-TRPM8 (residues 278-292 and 1090-1104; human) antibody was added to TRPMB-expressing or control membrane suspensions and rolled at 4°C for 16 h prior to use. In the case of the anti-TRPM8 (residues 656-680; rat) antibody, the antigenic peptide was available, so aliquots of the antibody were preincubated with the peptide (dissolved at a stock concentration of 1mg/180 µl in PBS) and used at a ratio of 5 µg peptide: 1 µg antibody, by rolling at 4°C for 16 h. Control aliquots were treated with PBS alone.

### Immunohistochemistry

DRGs and spinal cord from lumbar segments L5/6, ipsilateral or contralateral to CCI or from naive or rhizotomised animals were taken. Tissue was snap frozen in liquid nitrogen and embedded in OCT (Cell Path plc. Powys. Wales, UK). Cryostat sections (15µm) were cut and thaw-mounted on poly-L-lysine slides (Merck-BDH). Slides were blocked in 10% normal goat serum, 0.1M PBS, pH 7.4, 0.2% Triton X-100, 4% fish skin gelatin for 1 h at room temperature and incubated overnight with appropriate primary antibodies in buffer (0.1 M PBS, pH 7.4, 0.2% Triton X-100, 4% normal goat serum, 4% fish skin gelatin). Antibodies used were: rabbit polyclonal anti-TRPM8, raised against residues 656-680 (rat) (Phoenix Peptides, 1:450), mouse monoclonal anti-NF-200 (Sigma, 1:1000), mouse monoclonal anti-peripherin (Chemicon, 1:250), mouse monoclonal anti-NeuN (Chemicon, 1:500). In experiments to assess specificity of the TRPM8 antibody used for immunohistochemistry, antibody aliquots were pre-incubated with the antigenic peptide or PBS control, as above. Slides were washed, incubated with appropriate fluorescent secondary antibodies in the same buffer excluding Triton X-100, (goat anti-rabbit AlexaFluor 568 1:750, goat anti-mouse AlexaFluor 488, 1:500, Molecular Probes, OR, USA), washed, stained with To-Pro nuclear stain (1:1000, To-Pro-3-iodide, Molecular Probes), coverslipped and sealed. Control sections were processed as above omitting the primary antisera. Sections were visualised using a Leica TCSNT confocal microscope (Leica Microsystems GMBH, Germany. Image analysis was performed with Image Tool software (UTHSCSA Image Tool Version 3.0) and illustrations prepared using Adobe Photoshop 7.0. Cell counts were performed on five to eight randomly selected DRG sections (separation of 100 µm) from each of three animals in each group, and only neurons with clear nuclei were counted. Results were expressed as the proportion of TRPM8-labelled cells per total number of peripherin/NF-200 labelled cells from all sections.

### Results

### Reversal of nerve injury-induced reflex sensitisation by peripheral activation of TRPM8 channels

In order to model potential clinical usage, we administered icilin topically to the paws by placing rats with chronic constriction injury (CCI) to sciatic nerve in a bath with 1 cm deep drug solution, kept at 30°C to avoid any effects on local skin temperature. After 5 min, icilin (80µM), but not vehicle (0.2% dimethylformamide in water), caused striking reversal of CCI-induced behavioural reflex sensitisation to thermal and mechanical stimuli (Fig 1A). Concentration-dependent effects were observed from 2.5µM up to a maximum of 500µM, with no effect on contralateral or naive responses (Fig 1B). At much higher concentrations of icilin, we observed the beginning of a trend towards increasing reflex sensitivity, ipsilateral and contralateral to CCI and in naives (Table 1), that was statistically significant in mechanical and (after a delay) in thermal tests, only at the highest concentration tested, 5 mM.

**Table 1: Analgesic effects of topically administered icilin against neuropathic sensitisation revert at very high concentrations to a general nociceptive effect**

| **Reflex response sensitivity at different times after icilin application to paw Thermal PWL (s)** | | | | | | |
|---|---|---|---|---|---|---|
| **Drug Concentration (µM)** | **Naïve** | | **CCI Ipsi** | | **CCI Con** | |
| **Pre-drug Baseline** | 15.5 ± 0.2 | | 9.7 ± 0.2 | | 16.4 ± 0.2 | |

| | **15 mins** | **50 mins** | **15 mins** | **50 mins** | **15 mins** | **50 mins** |
|---|---|---|---|---|---|---|
| **0 (vehicle)** | 15.1 ± 1.2 | 14.9 ± 0.8 | 9.8 ± 1.1 | 9.7 ± 0.9 | 15.2 ± 0.6 | 15.1 ± 0.7 |
| **1000** | 15.1 ± 1.2 | 14.8 ± 0.9 | 16.4 ± 0.8† | 8.6 ± 0.2 | 16.1 ± 0.5 | 15.2 ± 0.4 |
| **2500** | 14.9 ± 0.3 | 13.2 ± 0.8 | 16.4 ± 0.9† | 8.4 ± 0.6 | 16.8 ± 0.9 | 15.3 ± 0.7 |
| **5000** | 15.3 ± 0.5 | 13.1 ± 0.7§ | 16.7 ± 1.0† | 7.2 ± 0.6§ | 16.1 ± 0.9 | 13.4 ± 0.7§ |

| **Mechanical PWT (mN/mm²)** | | | | | | |
|---|---|---|---|---|---|---|
| | **Naïve** | | **CCI Ipsi** | | **CCI** Con | |
| **Pre-drug Baseline** | 4830.6 ± 0.0 | | 805.7 ± 26.3 | | 4793.6 ± 37.5 | |

| | **15 mins** | **50 mins** | **15 mins** | **50 mins** | **15 mins** | **50 mins** |
|---|---|---|---|---|---|---|
| **0 (vehicle)** | 4830.6 ± 0.0 | 4830.6 ± 0.0 | 805.7 ± 26.3 | 805.7 ± 26.3 | 4830.6 ± 0.0 | 4830.6 ± 0.0 |
| **1000** | 4830.6 ± 0.0 | 4830.6 ± 0.0 | 702.8 ± 185.3 | 702.8 ± 185.3 | 4530.1 ± 300.5 | 4530.1 ± 300.5 |
| **2500** | 4830.6 ± 0.0 | 4830.6 ± 0.0 | 651.9 ± 193.0 | 651.9 ± 193.0 | 4454.9 ± 245.9 | 4454.9 ± 245.9 |
| **5000** | 3828.6 ± 316.8§ | 4830.6 ± 0.0 | 412.2 ± 24.3§ | 736.0 ± 26.3 | 2040.2 ± 115.2§ | 4830.6 ± 0.0 |

Significant effects of drug on reflex responses are indicated: † denotes significant increase from baseline indicating analgesic effect of icilin, § denotes significant decrease from baseline values indicating hyperalgesic effects.

This effect was distinct from the prominent analgesia at low concentrations of icilin as it was not specific to a sensitised state and may be due to weak interaction with other targets or non-specific actions. Specific reversal of sensitised responses was also caused by another selective TRPM8 activator, (-)-menthol (4mM). The stereoisomers isomenthol and (+)-menthol, which are several fold less potent agonists of TRPM8^{[44,45]} also produced reversal of sensitised responses at concentrations of 8mM and 16mM (Fig 1C),

Icilin is expected to activate TRPM8-containing afferents, so we recorded firing activity in saphenous nerve afferents following topical application of icilin (Fig 1D). The nerve was dissected to produce small-number preparations of fine afferents, with conduction velocities of up to 2.6ms⁻¹ (representing C- and Aδ-fibre afferents^{[46]}). Icilin (200µM) applied to the receptive field on the hindlimb caused a significant increase in firing frequency in 21.6% (40 out of 185) of recorded fine afferents with a mean 7-fold increase in firing frequency from baseline of 4.5 ± 2.5 Hz to 31.6 ± 3.4 Hz, mean time to peak effect of 3.3 ± 0.5 mins. Icilin did not produce rapid desensitisation, agreeing with some^{[47]}, but not other reports^{[48]}. Recovery was consistently observed. Similar results were obtained from both hairy and glabrous skin. Large myelinated mechanoreceptors (conduction velocities 6.8-15ms⁻¹, n=43) were unaffected.

Consistent with a TRPM8-mediated mechanism, paw immersion at 16-20°C for 5 min also produced statistically significant mechanical analgesia (Fig 1E). Recordings from a subcutaneous thermistor probe showed that deep skin temperatures were 0.5°C above bath temperatures after 5 min in similar conditions. This temperature is in the range expected to activate TRPM8, and stimulate innocuous cool-sensitive fibres. Immersion temperatures below 14°C (in the range where other cold sensors, in addition to TRPM8, are also likely to be activated) elicited active nociceptive withdrawal reflexes limited to the period of hindpaw immersion, in agreement with the known temperature activation range for nociceptive cold fibres^{[49]}.

### Localisation of TRPM8 in afferents and superficial dorsal horn: increased expression following nerve injury.

The presence and localisation of TRPM8 in DRG and spinal cord were investigated by immunoblotting and immunohistochemistry. Following rapid homogenisation of DRGs in Laemmli lysis buffer and SDS-PAGE, immunoblots probed with a rabbit polyclonal antibody raised to TRPM8 residues 278-292 and 1090-1104 (human)^{[50]}, showed a single, strong band at approximately 128 kDa (the predicted molecular weight of TRPM8), with faint bands observed at approximately 170, 60 and 50 kDa (Fig 2A). Both antigen-preabsorbtion and antisense-knockdown controls were consistent with specificity of this antibody in recognition of TRPM8 at approximately 128 kDa under the conditions used. Pre-incubation of the antibody with membranes from COS7 cells transfected with TRPM8 expression plasmid abolished the band at 128kDa, whereas sham preabsorbtion with membranes from cells with empty vector did not (Fig 2A). Correspondingly, intrathecal delivery of TRPM8 antisense oligonucleotide to naive (non-CCI) rats over 5 days, also resulted in almost complete knockdown of the 128kDa band (Fig 2A), whereas mis-sense control oligonucleotide was ineffective (see below). Intrathecally delivered fluorescent-labelled oligonucleotides have been shown to effectively penetrate the DRG, as soon as 4 hours after initial delivery^{[51]}. The faint bands, at 50 and 60 kDa at least, remained present in each case and so are likely to represent non-specific interactions of the antibody under these conditions. As further controls, we showed that TRPV1 immunoreactivity was unaffected by treatment with the TRPM8 antisense reagent and that the housekeeping enzyme GAPDH (36kDa) was evenly present in each lane (Fig 2A). Following nerve injury, there was a marked increase in expression of the 128kDa TRPM8-immunoreactive band specifically in ipsilateral, but not contralateral DRG (Fig 2A), while immunoreactivity for GAPDH was unaltered. Densitometric ratios for TRPM8 expression as percentage of GAPDH were 80.7 ± 4.1 ipsilateral to CCI, which was significantly greater than that seen contralateral to CCI (49.3 ± 3.2) and in naive DRG (50.7 ± 2.7), (means ± SEM, n=5-6). L4-5 spinal cord extracts showed that TRPM8 immunoreactivity was present centrally, and after preparation of a crude particulate fraction (centrifugation at 11,000g for 45 min) these also showed consistent increases in expression ipsilateral to injury. Densitometric values ipsilateral to CCI were 198 ± 6.7% of those in naives (p<0.05; means ± SEM, n=5), whereas contralateral values were 125 ± 7.1% (p>0.05).

Immunohistochemistry was carried out using a rabbit polyclonal antibody raised to TRPM8 residues 656-680 (rat)^{[52]} the specificity of which was addressed by antigen preabsorbtion and antisense knockdown controls. The labelling observed in a discrete subpopulation of DRG cells was abolished following pre-incubation with the peptide antigen (no positive cells seen, counted over twelve 500µm² sections, compared with a mean of 5.3 ± 0.4 TRPM8-positive cells per 500 µm² DRG section with sham treatment of antibody, and 5.1 ± 0.5 TRPM8-positive cells per 500 µm² DRG section with untreated antibody, counted over 12 sections each). In immunoblots, the antibody also labelled a single band at approximately 128kDa in naive DRG tissue that was abolished either by preabsorbtion with the peptide antigen or by prior 5 day intrathecal infusion of TRPM8 antisense (Fig 2B). Immunohistochemical staining in spinal cord showed that TRPM8 in spinal cord was largely expressed in superficial dorsal horn, like the C-fibre marker, peripherin (Fig 2C) and that following dorsal rhizotomy (L2-6), the vast majority of TRPM8 (and peripherin) immunoreactivity was lost ipsilaterally (reductions of approximately 80-90%) suggesting that spinal TRPM8 originates largely from afferents. In confirmation of the immunoblot findings, levels of TRPM8-like immunoreactivity were increased in dorsal horn ipsilateral to injury (by approximately 70-80%), but retained a similar distribution to that in naive animals (Fig 2D).

To establish whether the increases in afferent TRPM8 expression occurred in specific subpopulations of DRG cells, we investigated TRPM8 colocalisation with markers of myelinated afferents (neurofilament-200; NF-200^{[53]}) and unmyelinated afferents (peripherin^{[40]}), In naive rats, TRPM8 immunoreactivity was largely confined to a subpopulation of unmyelinated DRG cells (8.3 ± 0.2% of peripherin-positive cells; 34 of 408 cells) and only minimally expressed in myelinated, NF-200-positive cells (1.3 ± 0.5%; 6 of 445 cells). However, following CCI, TRPM8 expression was significantly increased ipsilaterally in both NF-200- and peripherin-positive cells to 7.9 ± 1.2% (31 of 390 cells) and 15.5 ± 0.8% (64 of 412 cells), respectively. Corresponding contralateral values were unaltered from naives at 2.0 ± 0.4% (14 of 346 cells) and 9.2 ± 0.4% (42 of 452 cells) (Fig 2 E, F). Data taken from 3 CCI and 3 naive animals, counted across 15 - 21 sections. The additional TRPM8-expressing NF-200-positive cells were small (average diameter, 19.7 ± 0.8µm), presumed Aδ myelinated neurons^{[53]}. There were no significant differences in the diameters of NF-200- or peripherin-positive cells or in the numbers of NF-200- or peripherin-positive DRG neurons per section.

### Molecular identification of TRPM8 as the mediator of icilin-induced analgesia.

To define the specific involvement of TRPM8 in icilin analgesia we further utilised the antisense oligonucleotide knockdown strategy. TRPM8 antisense or mismatched control oligonucleotides were delivered intrathecally over 13 days to parallel the sensitisation developing following CCI. The development of CCI-induced behavioural reflex sensitisation was unaffected, including thermal hyperalgesia and mechanical allodynia (Fig 3A, B) and cold allodynia (control CCI animals showed elevation of the paw ipsilateral to nerve injury out of 4°C water for 8.1 ± 0.5 s at peak, 9-11 days after surgery, whereas corresponding values in antisense-treated CCI animals were 7.6 ± 0.6s). In contrast, the reversal of neuropathic reflex sensitisation produced normally by 80µM icilin applied to the paws (Fig 1A) was abolished by treatment with antisense (Fig 3A), but not mis-sense (Fig 3B) reagents. The mean ± SEM % reversals of ipsilateral sensitisation over 10-25 minutes after icilin treatment in antisense- and mis-sense-treated animals were 7.7 ± 7.4% and 82.8 ± 6.9%, respectively for PWL, and 9.4 ± 8.2% and 58.7 ± 8.2% for PWT; with mis-sense-treated, but not antisense-treated animals, retaining significant effects of icilin (p<0.05). When antisense osmotic pumps were depleted, but animals were still neuropathic (18 days following surgery for insertion of 14 day minipumps and CCI), responses to icilin were restored to 83.7 ± 10.1% reversal of sensitisation for PWL, and 54.0 ± 7.2% for PWT. Effectiveness of knockdown was assessed by SDS-PAGE/immunoblotting. Expression of the 128kDa TRPM8-immunoreactive band in both ipsilateral and contralateral DRGs was greatly reduced by the antisense reagent and the increase in TRPM8 expression normally seen ipsilateral to nerve injury was prevented (Fig 3C). The mis-sense reagent had no effect (Fig 3C) showing TRPM8 expression similar to that in untreated CCI animals (Fig 2A). In mis-sense-treated animals, TRPM8:GAPDH ratios were 77.9 ± 2.0% ipsilateral to CCI and 52.9 ± 2.1% contralateral, similar to corresponding control values (Fig 2A and above), whereas in antisense-treated animals, values were much lower (19.8 ± 2.2% and 14.9 ± 2.1%, respectively, means ± SEM, n=5).

To confirm that antisense knockdown of TRPM8 resulted in associated functional changes in afferents, saphenous nerve recordings were made from naive animals receiving intrathecal delivery of TRPM8 antisense or mis-sense oligonucleotides, 4-5 days after insertion of pump. The increase in firing frequency evoked by topical icilin (200µM) was strongly reduced in animals receiving antisense. Only 3 out of 34 recorded slowly conducting fibres (8.8%) showed a partial activation in response to drug, increasing firing by approximately 2-fold from a baseline of 5.8 ± 1.4 to 12.7 ± 0.6 Hz, compared with the 7-fold increase observed in over 20% of fine afferents in naive animals. In contrast, mis-sense animals showed a 7-fold increase in firing frequency in 25% of 40 recorded fibres from a baseline of 3.3 ± 0.7 to 23.1 ± 3.2 Hz, similar to results from naïve animals. Similarly, in mis-sense-treated animals, topical (-)-menthol (4mM) produced an approximately 8-fold increase in mean firing frequency (from 4.5 ± 2.9 to 38.9 ± 7.6Hz), activating 20% of fibres (35 identified afferents recorded). This compared with no obviously activated afferents in antisense-treated animals (mean firing frequency 4.0 ± 1.8Hz at background, 4.8 ± 1.9 Hz post-drug application, 28 identified afferents recorded). However, TRPM8 antisense treatment did not alter the effect of topically applied resiniferatoxin (1mM), a potent TRPV1 agonist, acting as a control. In antisense-treated animals, resiniferatoxin evoked a 6-fold increase in firing frequency in activated afferents (from 4.5 ± 0.7 baseline to 25.9 ± 1.8 Hz at peak response, 16 afferents activated out of 28 recorded), which was similar to responses in mis-sense-treated animals (showing a mean 5-fold change in firing frequency from 4.6 ± 2.8 to 24.8 ± 3.1Hz).

### Central intrathecal administration of TRPM8 activators also inhibits neuropathic sensitisation.

Since TRPM8 is present on central terminals of primary sensory neurons as well as their peripheral terminals (Fig 2B,C,^{[54,55]}), we investigated whether intrathecal application of TRPM8 activators near to the central terminals would also produce analgesia. Intrathecal injection of icilin (10nmol) produced robust reversal of CCI-induced behavioural reflex sensitisation in thermal and mechanical tests (Fig 4A, p<0.05 for up to 55 min). Intrathecal injection of (-)-menthol (200nmol) in CCI rats also caused a significant reversal of the sensitised responses, lasting 35-40 min (Fig 4B). Due to the higher potency and efficacy of icilin at TRPM8^{[15]}, further experiments mainly utilised icilin as the representative TRPM8 activator. Icilin produced dose-dependent analgesic effects restricted to the nerve injury side in both thermal and mechanical tests that were statistically significant by 0.125nmol and increased to almost complete reversal of sensitisation by 10nmol. Non-linear curve-fitting indicated that maximal effects of icilin were similar for PWL and PWT (91.6 ± 9.9 and 82.6 ± 6.8% reversal of sensitisation, respectively), as were EC₅₀ values (dose for 50% of maximal effect; 0.17 ± 0.02 and 0.31 ± 0.02 nmol, respectively).

In complete contrast to the effects of TRPM8 activators, the TRPA1 activator, cinnamaldehyde^{[20]} (75nmol injected intrathecally) significantly increased reflex responsiveness in thermal and mechanical tests and was effective contralateral as well as ipsilateral to nerve injury (Fig 4C). The sensitising effects of cinnamaldehyde were prevented by co-injection of Ruthenium Red (0.25nmol), which can block TRPA1 channels^{[22,58]}, whereas the analgesic effect of intrathecally-injected icilin was unaffected (Fig 4D). Sensitising effects of cinnamaldehyde were also seen in naive animals with a 36.9 ± 7.9% reduction in PWL and a 41.9 ± 6.7% reduction in PWT. Similar effects were produced by two further TRPA1 activators^{[57,58]} allicin (25nmol), where corresponding reductions were 33.2 ± 5.9% and 20.7 ± 8.2%, respectively and diallyl disulphide (50nmol) with equivalent values of 25.9 ± 7.0% and 28.5 ± 6.2% (means ± SEM, n=3-6). In contrast, the TRPM8 activators, icilin (10nmol) and (-)-menthol (200nmol) were without effect in naïve animals (data not shown). Topical application of cinnamaldehyde (1.5mM) also produced bilateral sensitisation of behavioural reflexes in I animals (mean decrease of 32.0 ± 8.6% in PWL and 20.2 ± 8.2% in PWT, p<0.05, n=6). This corresponds to the licking and shaking behaviour as well as decrease in PWL reported following intraplantar injection of cinnamaldehyde^{[20]}.

In further experiments, we investigated whether the sensitised pain behaviours caused by TRPA1 activators or other pain models were susceptible to icilin-induced analgesia. Sensitisation caused by intrathecal or topical cinnamaldehyde was markedly attenuated by intrathecal icilin (Table 2).

**Table 2: Reversal of sensitisation by intrathecal icilin administration in different pain models.**

| **Pain Model** | **PWL (s) difference from control (no drug)** | **PWL (s) difference from control (+ icilin)** | **Mean % reversal of thermal sensitisation (after 15-30mins)** | **PWT (mN/mm²) difference from control (no drug)** | **PWT (mN/mm²) difference from control (+ icilin)** | **Mean % reversal of mechanical sensitisation (after 15-30mins)** |
|---|---|---|---|---|---|---|
| **CCI** | 5.9 ± 0.9* | 0.9 ± 1.2 † | 84.7 ± 6.0 | 3347.4 ± 126.3 * | 775.4 ± 467.7 † | 76.8 ± 5.3 |
| **CFA** | 4.7 ± 0.9 * | 1.6 ± 1.6 † | 66.0 ± 7.5 | 2975.6 ± 245.5 * | 1707.6 ± 149.9 † | 42.6 ± 2.8 |
| **Lysolecithin** | 7.8 ± 1.0* | 2.4 ± 1.2 † | 69.2 ± 5.1 | 3003.8 ± 182.4 * | 1102.5 ± 422.5 † | 63.3 ± 6.4 |
| **Cinnamaldehyde (intrathecal)** | 5.9 ± 0.9 * | 0.5 ± 1.8 † | 91.5 ± 4.1 | 2022.6 ± 375.4 * | 150.3 ± 125.2 † | 92.6 ± 2.8 |
| **Cinnamaldehyde (topical)** | 5.0 ± 1.5 * | 0.4 ± 1.6 † | 92.0 ± 6.1 | 1002.0 ± 316.8 * | 0.0 ± 0.0 † | 100.0 ± 0.0 |

| | | | | | | |
|---|---|---|---|---|---|---|
| (vi) Significant ipsilateral-contralateral differences in surgical pain models or, in the case of cinnamaldehyde-induced responses, differences from prior baseline are indicated (*, p<0.05). Significant icilin (10nmol, i.t.)-induced reversal of sensitisation is indicated (†, p<0.05). | | | | | | |

The effect of topical cinnamaldehyde was additionally reversed by topical icilin (200µM, data not shown). Sensitisation caused by focal demyelination of the sciatic nerve^{[40]} or intraplantar injection of Complete Freund's Adjuvant (CFA) was also significantly inhibited by intrathecal icilin (Table 2).

### Central mechanism of icilin reversal of neuropathic sensitisation involves mGlu group II/III receptors.

Since topical icilin increases activity in fine afferents (Fig 1D) and both intrathecal and topical icilin reverse nerve injury-induced sensitisation, centrally-mediated events are likely to be important in icilin action. Icilin-responsive afferents are expected to release glutamate, so we hypothesised that inhibitory glutamate receptors in dorsal horn might underlie icilin-induced analgesia. Group II/III mGluRs could subserve such a role since they are antinociceptive in models of inflammatory, neuropathic and acute pain^{[29,30,31]} and inhibit transmission between primary afferent and spinal cord neurons in sensitised states^{[32,33]}. Group II mGluRs are localised on primary afferent terminals in lamina II, particularly in small nociceptive afferents^{[27,60]}, although some are found post-synaptically and on glia^{[28]}. Group III mGluRs are also found pre-synaptically in the dorsal horn and are 45% co-expressed with either IB4 or Substance P (markers of small nociceptive neurons^{[59]}). To assess whether activation of Group II/III mGluRs might mimic icilin reversal of neuropathic sensitisation, we intrathecally injected the selective Group II or III mGluR agonists, 2R, 4R-APDC or ACPT-III and AP-4, respectively. 2R, 4R-APDC (15nmol) caused 72.1 ± 6.4% reversal of thermal and 56.0 ± 10.9% reversal of mechanical reflex sensitisation ipsilateral to CCI (15-30 min post-injection) with no effect on contralateral responses. ACPT-III and AP-4 (150nmol) each) also reversed thermal sensitisation (by 83.6 ± 6.3% and 60.8 ± 6.7%, respectively), as well as mechanical sensitisation (65.7 ± 11.4% and 60.7 ± 8.0%), again with no effects contralaterally (p<0.05 in each case). Furthermore, selective Group II and Group III mGluR antagonists, LY 341495 (5nmol, Fig 5A) and UBP 1112 (10nmol, Fig 5B) each prevented the effect of icilin (10nmol, Fig 4A). Similarly, the analgesia produced by intrathecal (-)-menthol (200nmol, Figure 4B) was reversed by intrathecal LY 341495 and UBP 1112. The mean percentage reversal of sensitisation over 20-30 minutes post-injection was 86.1 ± 8.1% for PWL and 80.6 ± 4.2% for PWT with (-)-menthol alone, 22.0 ± 6.9% for PWL and 7.1 ± 7.1% for PWT with menthol and LY 341495, and 9.2 ± 6.9% for PWL and 0.0 ± 0.0% for PWT with (-)-menthol and UBP 1112 (n=6). Neither LY 341495 nor UBP 1112 had any effects alone (data not shown), suggesting that Group II/III mGluRs show little tonic activation following CCI, but are specifically utilised downstream of icilin. In contrast, intrathecal co-administration of the µ-opioid receptor antagonist, naloxone (25nmol) with icilin had no effect (Fig 5C), indicating that icilin analgesia is opioid-independent. To avoid any possibility of non-specific drug interactions, we also administered icilin (200µM) topically, but the mGluR antagonists, intrathecally. Fig 5D shows that the icilin reversal of thermal and mechanical sensitisation in this case was again prevented by LY 341495 or UBP 1112. The analgesic effect of skin cooling to 16 °C (Fig 1E) was also prevented by intrathecally applied LY 341495 (5 nmol) or UBP 1112 (10 nmol). The mean percentage reversal of ipsilateral CCI-induced reductions in PWT caused by cooling was 0.0 ± 0.0% in the presence of either drug (n=5).

To confirm the analgesic effect of icilin at the level of single spinal cord neurons, we made in vivo extracellular recordings of large lamina I and III/IV neurons (that integrate nociceptive and non-nociceptive inputs). Topical administration of icilin (200µM) to the peripheral receptive field area ipsilateral to CCI caused inhibition of the elevated neuronal responses to motorised rotating brush (Fig 5E). In the 8 neurons out of 12 that were affected by icilin (2 in lamina I and 6 in laminae III/IV) brush-induced responses were reduced to 37.4 ± 5.5%, p<0.001). Vehicle had no effect. Contralateral neurons were unaffected (111.9 ± 8.9% of control; n=6). As an example of one of the Group II/III mGluR antagonists investigated on reflexes, UBP 1112 was ionophoresed in the vicinity of recorded dorsal horn neurons at currents of 20-60nA. UBP 1112 reversed the effect of icilin; the brush-induced firing rate reverted to 80.2 ± 9.3% of control values (Fig 5E), but UBP 1112 had no effect alone, nor did saline current controls.

Since some Group II/III mGluRs may be post-synaptic, we asked whether icilin could reverse the additional sensitisation of behavioural reflex responsiveness caused by intrathecally applied NMDA in CCI animals. Icilin (10 nmol) clearly attenuated the additional ipsilateral sensitisation induced by 3.75 nmol of NMDA plus 0.75 nmol of its co-agonist site activator, ACPC, injected intrathecally. Ipsilateral PWL values in thermal tests were 10.1 ± 0.6 s at baseline and decreased to 7.9 ± 0.3s (15-30 minutes after injection of NMDA/ACPC), but increased to 14.9 ± 0.6 s in the additional presence of icilin. Contralateral values were unaltered by icilin or NMDA/ACPC. Ipsilateral PWT values in mechanical tests were 1504.2 ± 105.3 mN/mm² at baseline, 891.6 ± 27.3 mN/mm² after injection of NMDA/ACPC and 3482.7 ± 174.3 mN/mm² with co-injection of icilin (means ± SEM, n=6). Contralateral values again were unaltered. A component of the central events elicited by icilin may therefore be post-synaptic, although it is important to note that functional NMDA receptors may also be present on afferent terminals^{[61]}.

### Discussion

Little is known of the mechanism underlying cooling-induced analgesia, but a number of cool-sensitive ion channels, including TRPM8, have recently been identified in somatosensory afferents^{[31]} We now show that TRPM8 activation reverses nerve injury-induced hypersensitivity. TRPM8 can be activated by menthol^{[15.16]}, which is analgesic in hot plate and acetic acid writhing tests^{[11]}, although menthol can produce pain at very high doses[^{62,63]}. Here, either topical or intrathecal application of (-)-menthol produced behavioural analgesia in the CCI model of neuropathic pain, most likely by activation of TRPM8. Similar effects were seen with another TRPM8 activator, icilin^{[15]}. As with menthol, very high doses of icilin were found to cause a generalised increase in sensitisation, affecting CCI animals bilaterally and naïve animals in a similar fashion (Table 1). Importantly, analgesic effects of icilin were seen at 200-fold lower concentrations than those causing non-specific sensory changes. Specific involvement of TRPM8 in the reversal of neuropathic sensitisation was confirmed by the abrogation of icilin analgesia following intrathecal infusion of a TRPM8 antisense oligonucleotide to knock-down. TRPM8 expression. Furthermore, the analgesic profile was mimicked by cutaneous cooling to 20-16°C, a range activating the TRPM8 channel^{[14]}. Icilin and menthol were applied cutaneously in solution at 30°C, so any possible drug effects on skin temperature were avoided. TRPM8 antisense had no effect alone on CCI-induced sensitised responses to noxious heat, mechanical stimuli or intense cold (Fig 3), similar to observations made in an alternative neuropathic pain model^{[26]}. A role for the TRPA1 channel in analgesia seems unlikely since selective TRPA1 activators, cinnamaldehyde, allicin and diallyl disulphide, caused contrasting sensitisation/hyperalgesia not only after CCI, but also in naïve animals. The analgesic effects of icilin were only seen in the sensitised pain state, but were not restricted to nerve injury, since sensitisation due to peripheral inflammation, afferent demyelination and TRPA1 activation was also reduced. The significant behavioural and electrophysiological effects of topically applied icilin demonstrate that icilin can cross the skin sufficiently to excite peripheral afferents, and point to the likely clinical utility of this or related drugs.

The precise identity of the TRPM8-containing cool-responsive afferents is not clear. Subpopulations of Aδ- and C-fibres are responsive to different ranges of cool temperatures ∼15-30°C, and <15°C^{[55,37]}. Innocuous cooling (15-30°C) activates a subpopulation of Aδ- and C-fibres in primates, but almost solely unmyelinated fibres in rodents^{[64]}. In contrast, intense noxious cold is signalled by unmyelinated polymodal nociceptors, which also respond to heat and mechanical stimuli^{[49]}. The TRPM8 activator menthol activates cool-sensitive fibres, and sensitises stimulus-induced responses in the range 20-30 °C^{[65,66]}. Studies of TRPM8 in vitro identify this channel as a likely transducer of moderate cool temperatures^{[19]}. In DRG and trigeminal cultures, responses to menthol, cooling (15-30°C) and TRPM8 mRNA expression all correlate closely^{[17,18,19]}. TRPM8 is expressed in 5-20% of DRG cell bodies that are small and presumed Aδ- or C-fibres^{[15,16]}, but not in large myelinated fibres. We observed that TRPM8 immunoreactivity is normally associated with a subpopulation of peripherin-positive C-fibres, but only minimally with NF-200-positive afferents, whereas high sensitivity cRNA hybridisation suggests the presence of some TRPM8 mRNA in up to 19% of NF-200-positive afferents^{[67]}. The capsaicin-and heat-sensitive TRPV1 channel, which contributes to thermal nociception and inflammatory sensitisation^{[12]}, is found in both peptidergic afferents (∼85%), and non-peptidergic (isolectin-B4, IB4-positive) cells in the rat^{[68]}. TRPM8 is not categorically associated with either peptidergic or IB4-positive afferents^{[15]} but is often present in those containing the NGF receptor, Trk A^{[67]}. Different groups have reported co-expression of mRNAs for TRPM8 and TRPV1 or menthol/capsaicin responsiveness of DRGs at 29-50%^{[15,19,60]} or close to zero^{[14,16,69]}. Overall it seems likely that TRPM8 is normally expressed in a distinct population of cool-responsive afferents and possibly also to an extent in some nociceptors. Our findings further identified increased expression in peripherin-positive C-fibres, but induction in small NF-200-positive presumed Aδ-fibres^{[46]}, suggesting that plasticity in TRPM8 expression may participate in icilin analgesia in neuropathic pain. No changes in DRG expression of TRPM8 were reported in an alternative neuropathic pain model (ligation of L5 spinal nerve or indeed following CFA) ^{[25,26]}, suggesting that specific aspects of the particular model, such as the co-existence of injured and uninjured afferents in DRG following CCI, may be important in TRPM8 upregulation.

TRPA1, which has also been proposed as a cool receptor^{[14]} appears to play an entirely different role, eliciting reflex pain behaviours in naive animals, as well as increasing thermal and mechanical responsiveness in the neuropathic state. This may correspond to clinical observations after nerve injury in which moderately cool stimuli are perceived as painful^{[1]}. TRPA1 is present mainly in small cells in sensory ganglia^{[14,22} and may increase ipsilateral to nerve injury and inflammation^{[25,26]} Nerve-injury and inflammation-induced hyperalgesia to noxious cold (5°C) is reported to be decreased by antisense knockdown of TRPA1^{[25,26}. Correspondingly, mutant mice homozygous for targeted disruption of the TRPA1 gene show reduced reflex withdrawal responses to selective TRPA1 activators and reduced sensitisation of noxious heat and innocuous mechanical responses caused by these agents^{[23]}. However, the role of TRPA1 in noxious cold responses is disputed, with results from different lines of TRPA1^{-/-} animals showing either attenuated or unaltered coldplate withdrawal responses^{[23,24}. Thus the precise role of TRPA1 in cold sensation remains unclear, but here as in other studies TRPA1 clearly acts in a pro-nociceptive manner^{[20,23,57,58]}. Although icilin may interact with low potency at the TRPA1 channel^{[14]}, the analgesic profile of TRPM8 activators here is entirely different from the pro-nociceptive profile of TRPA1 activators. The extent of TRPMB/TRPA1 co-expression in afferents is reported to be minimal^{[14,67]}.

The analgesia induced by icilin and menthol and by skin cooling to 16°C was shown to be centrally-mediated and dependent on Group II/III mGluRs. The lack of effect of naloxone suggests independence from classical opioid analgesia. Furthermore, at the doses used, mGluR Group II/III antagonists selectively reversed icilin and menthol analgesia in sensitised responses, without any effects alone. Group II/III mGluRs are known to inhibit nociceptive responses^{[29,30,31,32,33]} and we showed that Group II/III mGluR agonists selectively inhibit sensitised responses in neuropathic pain. Both Group II and III mGluR subtypes are expressed in primary afferents, especially IB4-positive cells^{[27,59,60]}. Activation of Group II/III mGluRs can inhibit afferent-evoked potentials in dorsal horn^{[32]}, since Group II mGluRs are both pre- and post-synaptic at primary afferent synapses^{[28]}, whereas Group III mGluRs are largely presynaptic^{[69]}. Menthol is reported to increase mEPSC frequency at some synapses between DRG and dorsal horn neurons in culture and in slices^{[54,55]}, presumably corresponding to the activation of TRPM8-containing afferents (Fig 1D) and increased release of glutamate. Figure 6 shows a schematic outline of a model in which glutamate released from TRPM8-expressing afferents could mediate icilin-induced analgesia by acting on Group II/III mGluRs (located pre-synaptically on nociceptive afferents and possibly also post-synaptically) to result in attenuation of pain-related sensitisation (Fig 5E) and behavioural analgesia (Fig 5D).

### Conclusions

In summary, these novel findings show that both peripheral and central activation of TRPM8 can produce an analgesic effect that specifically reverses the sensitisation of behavioural reflexes elicited by peripheral nerve injury. This effect is produced by very low concentrations of topically applied TRPM8 activators, pointing to the likelihood of its ready utility in a clinical context. Other sensitised pain states, in addition to that induced by nerve injury, are similarly sensitive to reversal by TRPM8 activation, emphasising the likely value of TRPM8 activators and downstream central mediators of TRPM8 action, such as Group II/III mGluRs, as targets for the development of novel analgesics.

### References

1. Woolf, C.J., and Mannion, R.J. (1999). Neuropathic pain: aetiology, symptoms, mechanisms, and management. Lancet 353, 1959-1964.
2. Sprengell, C. (1735). Hippocrates: The Aphorisms of Hippocrates, and the sentences of Celsus; with explanations and references to the most considerable writers ... To which are added, aphorisms upon several distempers, not well distinguished by the ancients. Second edition, corrected and very much enlarged. Kt. London [s.n.].
3. Siegel, R.E. (1970). Galen on sense perception; his doctrines, observations and experiments on vision, hearing, smell, taste, touch and pain, and their historical sources. Basel: Karger.
4. Bini, G., Cruccu, G., Hagbarth, K.E., Schady, W., and Torebjork, E. (1984). Analgesic effect of vibration and cooling on pain induced by intraneural electrical stimulation. Pain 18, 239-248.
5. Sauls, J. (1999). Efficacy of cold for pain: fact or fallacy? Online J Knowl Synth Nurs 6, 8.
6. Wright, A. (1870). Oil of peppermint as a local anaesthetic. Lancet 2464, 726.
7. Blumenthal, M., Busse, W.R., Goldberg, A., Gruenwald, J., Hall, T., Riggins, C.W., Rister, R.S., editors. Klein, S., Rister, R.S., translators (1998). The Complete German Commission E Monographs-Therapeutic Guide to Herbal Medicines. Austin (TX): American Botanical Council; Boston: Integrative Medicine Communication.
8. Gobel, H., Schmidt, G. & Soyka, D. (1994). Effect of peppermint and eucalyptus oil preparations on neurophysiological and experimental algesimetric headache parameters. Cephalalgia 14, 228-234.
9. Davies, S.J., Harding, L.M., and Baranowski, A.P. (2002). A novel treatment of postherpetic neuralgia using peppermint oil. Clin J Pain 18, 200-202.
10. Green, B.G. & McAuliffe, B.L. (2000). Menthol desensitization of capsaicin irritation. Evidence of a short-term anti-nociceptive effect. Physiol Behav 68, 631-639.
11. Galeotti, N., Di Cesare Mannelli, L., Mazzanti, G., Bartolini, A. & Ghelardini, C. (2002). Menthol: a natural analgesic compound. Neurosci Lett 322, 145-148.
12. Caterina, M.J., Leffler, A., Malmberg, A.B., Martin, W.J., Trafton, J., Petersen-Zeitz, K.R., Koltzenburg, M., Basbaum, A.I., and Julius, D. (2000). Impaired nociception and pain sensation in mice lacking the capsaicin receptor. Science 288, 306-313.
13. McKemy, D. D. (2005). How cold is it? TRPM8 and TRPA1 in the molecular logic of cold sensation. Mol Pain 1, 16.
14. Story, G.M., Peier, A.M., Reeve, A.J., Eid, S.R., Mosbacher, J., Hricik, T.R., Earley, T.J., Hergarden, A.C., Andersson, D.A., Hwang, S.W. et al. (2003). ANKTM1, a TRP-like channel expressed in nociceptive neurons, is activated by cold temperatures. Cell 112, 819-829.
15. McKemy, D.D., Neuhausser, W.M. & Julius, D. (2002). Identification of a cold receptor reveals a general role for TRP channels in thermosensation. Nature 416, 52-58.
16. Peier, A.M., Moqrich, A., Hergarden, A.C., Reeve, A.J., Andersson, D.A., Story, G.M., Earley, T.J., Dragoni, I., McIntyre, P., Bevan, S., and Patapoutian, A. (2002). A TRP channel that senses cold stimuli and menthol. Cell 108, 705-715
17. Nealen, M.L., Gold, M.S., Thut, P.D. & Caterina, M.J. (2003).TRPM8 mRNA is expressed in a subset of cold-responsive trigeminal neurons from rat. J Neurophysiol 90, 515-520.
18. Thut, P.D., Wrigley, D., Gold, M.S. (2003). Cold transduction in rat trigeminal ganglia neurons in vitro. Neuroscience 119, 1071-1083.
19. Babes, A., Zorzon, D. & Reid, G. (2004). Two populations of cold-sensitive neurons in rat dorsal root ganglia and their modulation by nerve growth factor. Eur J Neurosci 20, 2276-2282.
20. Bandell, M., Story, G.M., Hwang, S.W., Viswanath, V., Eid, S.R., Petrus, M.J., Earley, T.J., and Patapoutian, A. (2004). Noxious cold ion channel TRPA1 is activated by pungent compounds and bradykinin. Neuron 41, 849-857.
21. Jordt, S.E., Bautista, D.M., Chuang, H.H., McKemy, D.D., Zygmunt, P.M., Hogestatt, E.D., Meng, I.D., and Julius, D. (2004). Mustard oils and cannabinoids excite sensory nerve fibres through the TRP channel ANKTM1. Nature 427, 260-265.
22. Nagata, K., Duggan, A., Kumar, G. & Garcia-Anoveros, J. (2005). Nociceptor and hair cell transducer properties of TRPA1, a channel for pain and hearing. J Neurosci 25, 4052-4061.
23. Bautista, D.M., Jordt, S.E., Nikai, T., Tsuruda, P.R., Read, A.J., Poblete, J., Yamoah, E.N., Basbaum, A.I., Julius, D. (2006). TRPA1 mediates the inflammatory actions of environmental irritants and proalgesic agents. Cell 124, 1269-1282
24. Kwan, K. Y., Allchome, A. J., Vollrath, M. A., Christensen, A. P., Zhang, D. S., Woolf, C. J., and Corey, D. P. (2006). TRPA1 contributes to cold, mechanical, and chemical nociception but is not essential for hair-cell transduction. Neuron 50, 277-289.
25. Obata, K., Katsura, H., Mizushima, T., Yamanaka, H., Kobayashi, K., Dai, Y., Fukuoka, T., Tokunaga, A., Tominaga, M., Noguchi, K. (2005). TRPA1 induced in sensory neurons contributes to cold hyperalgesia after inflammation and nerve injury. J Clin Invest 115, 2393-2401
26. Katsura, H., Obata, K., Mizushima, T., Yamanaka, H., Kobayashi, K., Dai, Y., Fukuoka, T., Tokunaga, A., Sakagami, M., Noguchi, K. (2006). Antisense knock down of TRPA1, but not TRPM8, alleviates cold hyperalgesia after spinal nerve ligation in rats. Experimental Neurology, IN PRESS.
27. Carlton, S.M., Hargett, G.L. & Coggeshall, R.E. (2001). Localization of metabotropic glutamate receptors 2/3 on primary afferent axons in the rat. Neuroscience 105, 957-969.
28. Tamaru, Y., Nomura, S., Mizuno, N. & Shigemoto, R. (2001). Distribution of metabotropic glutamate receptor mGluR3 in the mouse CNS: differential location relative to pre- and postsynaptic sites. Neuroscience 106, 481-503.
29. Fisher, K., Lefebvre, C. & Coderre, T.J. (2002). Antinociceptive effects following intrathecal pretreatment with selective metabotropic glutamate receptor compounds in a rat model of neuropathic pain. Pharmacol Biochem Behav 73, 411-418.
30. Simmons, R.M., Webster, A.A., Kalra, A.B. & Iyengar, S. (2002). Group II mGluR receptor agonists are effective in persistent and neuropathic pain models in rats. Pharmacol Biochem Behav 73, 419-427.
31. Chen, S.R. & Pan, H.L. (2005). Distinct roles of group III metabotropic glutamate receptors in control of nociception and dorsal horn neurons in normal and nerve-injured rats. J Pharmacol Exp Ther 312, 120-126.
32. Gerber, G., Zhong, J., Youn, D. & Randic, M. (2000). Group II and group III metabotropic glutamate receptor agonists depress synaptic transmission in the rat spinal cord dorsal horn. Neuroscience 100, 393-406.
33. Neugebauer, V., Chen, P.S. & Willis, W.D. (2000). Groups II and III metabotropic glutamate receptors differentially modulate brief and prolonged nociception in primate STT cells. J Neurophysiol 84, 2998-3009.
34. Carter, G.T., Jensen, M.P., Galer, B.S., Kraft, G.H., Crabtree, L.D., Beardsley, R.M., Abresch, R.T., Bird, T.D. (1998). Neuropathic pain in Charcot-Marie-tooth disease. Arch. Phys. Med. Rehabil 79, 1560-1564.
35. Aley, K.O., Reichling, D.B., Levine, J.D. (1996). Vincristine hyperalgesia in the rat: a model of painful vincristine neuropathy in humans. Neuroscience 73, 256-265.
36. Coleman RE. (1997). Skeletal complications of malignancy. Cancer 15, 80:1588-94.
37. Luger NM, Mach DB, Sevcik MA, Mantyh PW. (2005). Bone cancer pain: from model to mechanism to therapy. J Pain Symptom Manage. 29, S32-46.
38. Peters CM, Ghilardi JR, Keyser CP, Kubota K, Lindsay TH, Luger NM, Mach DB, Schwei MJ, Sevcik MA, Mantyh PW. (2005). Tumor-induced injury of primary afferent sensory nerve fibers in bone cancer pain. Exp Neurol, 193, 85-100.
39. Garry, E.M., Delaney, A., Blackburn-Munro, G., Dickinson, T., Moss, A., Nakalembe, I., Robertson, D.C., Rosie, R., Robberecht, P., Mitchell, R., and Fleetwood-Walker, S.M. (2005). Activation of p38 and p42/44 MAP kinase in neuropathic pain: Involvement of VPAC(2) and NK(2) receptors and mediation by spinal glia. Mol Cell Neurosci 30, 523-537.
40. Wallace, V.C., Cottrell, D.F., Brophy, P.J. & Fleetwood-Walker, S.M. (2003). Focal lysolecithin-induced demyelination of peripheral afferents results in neuropathic pain behavior that is attenuated by cannabinoids. J Neurosci 23, 3221-3233.
41. Soliman, A.C., Yu, J.S., Coderre, T.J. (2005). mGlu and NMDA receptor contributions to capsaicin-induced thermal and mechanical hypersensitivity. Neuropharmacology 48, 325-332.
42. Garry, E.M., Moss, A., Delaney, A., O'Neill, F., Blakemore, J., Bowen, J., Husi, H., Mitchell, R., Grant, S.G., Fleetwood-Walker, S.M. (2003). Neuropathic sensitization of □eurons□ral reflexes and spinal NMDA receptor/CaM kinase II interactions are disrupted in PSD-95 mutant mice. Curr Biol 18, 13:321-328.
43. Iggo, A. (1958). The electrophysiological identification of single nerve fibres, with particular reference to the slowest-conducting vagal afferent fibres in the cat. J Physiol 142, 110-126.
44. Behrendt, H.J., Germann, T., Gillen, C., Hatt, H., Jostock, R. (2004). Characterization of the mouse cold-menthol receptor TRPM8 and vanilloid receptor type-1 VR1 using a fluorometric imaging plate reader (FLIPR) assay. Br J Pharmacol 141 737-745.
45. Bandell, M., Dubin, A.E., Petrus, M.J., Orth, A., Mathur, J., Hwang, S.W., Patapoutian, A. (2006). High-throughput random mutagenesis screen reveals TRPM8 residues specifically required for activation by menthol. Nat Neurosci 9 493-500.
46. Harper, A.A. & Lawson, S.N. (1985). Conduction velocity is related to morphological cell type in rat dorsal root ganglion □eurons. J Physiol 359, 31-46.
47. Weil, A., Moore, S.E., Waite, N.J., Randall, A. & Gunthorpe, M.J. (2005). Conservation of functional and pharmacological properties in the distantly related temperature sensors TRVP1 and TRPM8. Mol Pharmacol 68, 518-527.
48. Chuang, H.H., Neuhausser, W.M. & Julius, D. (2004). The super-cooling agent icilin reveals a mechanism of coincidence detection by a temperature-sensitive TRP channel. Neuron 43, 859-869.
49. Georgopoulos, A.P. (1976). Functional properties of primary afferent units probably related to pain mechanisms in primate glabrous skin. J Neurophysiol. 39, 71-83.
50. Thebault, S., Lemonnier, L., Bidaux, G., Flourakis, M., Bavencoffe, A., Gordienko, D., Roudbaraki, M., Delcourt, P., Panchin, Y., Shuba, Y., et al (2005). Novel role of cold/menthol-sensitive transient receptor potential melastatine family member 8 (TRPM8) in the activation of store-operated channels in LNCaP human prostate cancer epithelial cells. J Biol Chem. 280, 39423-39435.
51. Lai, J., Gold, MS., Kim, C.S., Bian, D., Ossipov, M.H., Hunter, J.C., Porreca, F. (2002). Inhibition of neuropathic pain by decreased expression of the tetrodotoxin-resistant sodium channel, NaV1.8. Pain 95, 143-152.
52. Brauchi, S., Orio, P., Latorre, R. (2004). Clues to understanding cold sensation: thermodynamics and electrophysiological analysis of the cold receptor TRPM8. Proc Natl Acad Sci U S A 101, 15494-15499.
53. Lawson, S.N. & Waddell, P.J. (1991). Soma neurofilament immunoreactivity is related to cell size and fibre conduction velocity in rat primary sensory neurons. J Physiol 435, 41-63.
54. Baccei, M.L., Bardoni, R. & Fitzgerald, M. (2003). Development of nociceptive synaptic inputs to the neonatal rat dorsal horn: glutamate release by capsaicin and menthol. J Physiol 549, 231-242.
55. Tsuzuki, K., Xing, H., Ling, J. & Gu, J.G. (2004). Menthol-induced Ca2+ release from presynaptic Ca2+ stores potentiates sensory synaptic transmission. Journal of Neuroscience 24, 762-771.
56. Eccles, R. (1994). Menthol and related cooling compounds. J Pharm Pharmacol 46, 618-630.
57. Bautista, D.M., Movahed, P., Hinman, A., Axelsson, H.E., Sterner, O., Hogestatt, E.D., Julius, D., Jordt, S.E., and Zygmunt, P.M. (2005). Pungent products from garlic activate the sensory ion channel TRPA1. Proc Natl Acad Sci U S A 102, 12248-12252.
58. Macpherson, L.J., Geierstanger, B.H., Viswanath, V., Bandell, M., Eid, S.R., Hwang, S., and Patapoutian, A. (2005). The pungency of garlic: activation of TRPA1 and TRPV1 in response to allicin. Curr Biol 15, 929-934.
59. Li, H., Ohishi, H., Kinoshita, A., Shigemoto, R., Nomura, S., Mizuno, N. (1997). Localization of a metabotropic glutamate receptor, mGluR7, in axon terminals of presumed nociceptive, primary afferent fibers in the superficial layers of the spinal dorsal horn: an electron microscope study in the rat. Neurosci Lett 223, 153-156.
60. Jia, H., Rustioni, A., Valtschanoff, J. G. (1999). Metabotropic glutamate receptors in superficial laminae of the rat dorsal horn. J Comp Neurol 410, 627-642.
61. Liu, H., Wang, H., Sheng, M., Jan, L.Y., Jan, Y.N., and Basbaum, A.I. (1994). Evidence for presynaptic N-methyl-D-aspartate autoreceptors in the spinal cord dorsal horn. Proc Natl Acad Sci U S A 91, 8383-8387.
62. Wasner, G., Schattschneider, J., Binder, A. & Baron, R. (2004). Topical menthol-a human model for cold pain by activation and sensitization of C nociceptors. Brain 127, 1159-1171.
63. Namer B, Seifert F, Handwerker HO, Maihofner C (2005). TRPA1 and TRPM8 activation in humans: effects of cinnamaldehyde and menthol. Neuroreport 21, 16:955-959.
64. Iggo, A. (1969). Cutaneous thermoreceptors in primates and sub-primates. J Physiol 200, 403-430.
65. Hensel, H. & Zotterman, Y. (1951). The response of the cold receptors to constant cooling. Acta Physiol Scand 22, 96-105.
66. Schafer, K., Braun, H.A. & Isenberg, C. (1986). Effect of menthol on cold receptor activity. Analysis of receptor processes. J Gen Physiol 88, 757-776.
67. Kobayashi, K., Fukuoka, T., Obata, K., Yamanaka, H., Dai, Y., Tokunaga, A., Noguchi, K. (2005). Distinct expression of TRPM8, TRPA1, and TRPV1 mRNAs in rat primary afferent neurons with Aδ/C-fibers and colocalization with Trk receptors. J Comp Neurol 493, 596-606.
68. Tominaga, M., Caterina, M.J., Malmberg, A.B., Rosen, T.A., Gilbert, H., Skinner, K., Raumann, B.E., Basbaum, A.I., and Julius, D. (1998). The cloned capsaicin receptor integrates multiple pain-producing stimuli. Neuron 21, 531-543.
69. Okazawa, M., Inoue, W., Hori, A., Hosokawa, H., Matsumura, K., and Kobayashi, S. (2004). Noxious heat receptors present in cold-sensory cells in rats. Neurosci Lett 359, 33-36.

## Claims

1. A transient receptor potential (TRP) M8 cation channel activating agent selected from the group consisting of:
(i) icilin (1-(2'-hydroxyphenyl)-4-(3"-nitrophenyl)-1,2,3,6-tetrahydropyrimidin-2-one); and
(ii) menthol 1R,2S,5R-(5-methyl-2-[1-methylethyl] cyclohexanol);
(iii) icilin comprising a modification at the 2' position to include a member selected from the groups consisting of
(i) C₁-C₄ alkyloxy;
(ii) NO₂;
(iii) =O; and
(iv) NH₂;
(iv) methoxyicilin;
for use in inducing analgesia in a patient suffering from or experiencing chronic neuropathic pain.

2. Use of a transient receptor potential (TRP) M8 cation channel activating agent in the manufacture of a medicament for use in the induction of analgesia in a patient suffering from or experiencing chronic neuropathic pain, wherein the TRPM8 cation channel activating agent is selected from the group consisting of:
(i) icilin (1-(2'-hydroxyphenyl)-4-(3"-nitrophenyl)-1,2,3,6-tetrahydropyrimidin-2-one); and
(ii) menthol 1R,2S,5R-(5-methyl-2-[1-methylethyl] cyclohexanol);
(iii) icilin comprising a modification at the 2' position to include a member selected from the groups consisting of
(v) C₁-C₄ alkyloxy;
(vi) NO₂;
(vii) =O; and
(viii) NH₂;
(iv) methoxyicilin.

3. TRPM8 activating agent for the use of claim 1 or the use according to claim 2, wherein the TRPM8 activating agent is 1R,2S,5R-(5-methyl-2-[1-methylethyl]-cyclohexanol).

4. TRPM8 activating agent for use or the use according to any preceding claim, wherein the chronic neuropathic pain is selected from the following pain states:
(i) Trauma-induced neuropathic pain.
(ii) Demyelination-induced pain.
(iii) Inflammatory pain states.
(iv) Phantom-limb pain
(v) Neuropathic pain linked to cancer
(vi) Chemotherapy-induced neuropathy
(vii) Back pain
(vii) Bone pain and cancer-induced bone pain

5. TRPM8 activating agent for use or the use according to any preceding claim, wherein the medicament is intended to be administered at a peripheral site, topically, intrathecally, as an epidural, transdermally or via a transdermal delivery device.

6. TRPM8 activating agent for use or the use according to any preceding claim wherein the medicament is intended to be administered with another compound or compounds.

## Patentansprüche

1. Mittel zum Aktivieren von Transient-Rezeptor-Potential (TRP) M8-Kationenkanälen, das aus der Gruppe ausgewählt ist, die besteht aus:
(i) Icilin, (1-(2'-Hydroxyphenyl)-4-(3"-nitrophenyl)-1,2,3,6-tetrahydropyrimidin-2-on); und
(ii) Menthol, 1R,2S,5R-(5-Methyl-2-[1-methylethyl]cyclohexanol);
(iii) Icilin, umfassend eine Modifikation an der 2'-Position, um ein Mitglied aufzunehmen, das aus den Gruppen ausgewählt, die bestehen aus:
(i) C₁-C₄-Alkyloxy;
(ii) NO₂;
(iii) =O; und
(iv) NH₂;
(iv) Methoxyicilin;
zur Verwendung beim Induzieren von Analgesie in einem Patienten, der an chronischem neuropathischem Schmerz leidet oder solchen fühlt.

2. Verwendung eines Mittels zum Aktivieren von Transient-Rezeptor-Potential (TRP) M8-Kationenkanälen bei der Herstellung eines Medikaments zur Verwendung bei der Induktion von Analgesie in einem Patienten, der an chronischem neuropathischem Schmerz leidet oder einen solchen fühlt, wobei das Mittel zum Aktivieren vom TRPM8-Kationenkälen aus der Gruppe ausgewählt ist, die besteht aus:
(i) Icilin, (1-(2'-Hydroxyphenyl)-4-(3"-nitrophenyl)-1,2,3,6-tetrahydropyrimidin-2-on); und
(ii) Menthol, 1R,2S,5R-(5-Methyl-2-[1-methylethyl]cyclohexanol);
(iii) Icilin, umfassend eine Modifikation an der 2'-Position, um ein Mitglied aufzunehmen, das aus den Gruppen ausgewählt, die bestehen aus:
(v) C₁-C₄-Alkyloxy;
(vi) NO₂;
(vii) =O; und
(viii) NH₂;
(iv) Methoxyicilin.

3. TRPM8-Aktivierungsmittel zur Verwendung nach Anspruch 1 oder Verwendung nach Anspruch 2, wobei das TRPM8-Aktivierungsmittel 1R,2S,5R-(5-Methyl-2-[1-methylethyl]-cyclohexanol) ist.

4. TRPM8-Aktivierungsmittel zur Verwendung oder Verwendung nach einem der vorhergehenden Ansprüche, wobei der chronische neuropathische Schmerz aus den folgenden Schmerzzuständen ausgewählt ist:
(i) traumainduziertem neuropathischem Schmerz
(ii) demyelinisationsinduziertem Schmerz
(iii) entzündlichem Schmerzzuständen
(iv) Phantomgliederschmerz
(v) neuropathischem Schmerz in Verbindung mit Krebs
(vi) chemotherapieinduzierter Neuropathie
(vii) Rückenschmerz
(viii) Knochenschmerz und krebsinduziertem Knochenschmerz

5. TRPM8-Aktivierungsmittel zur Verwendung oder Verwendung nach einem der vorhergehenden Ansprüche, wobei das Medikament an einer peripheren Stelle, topisch, intrathekal, als Epiduralanästhesie, transdermal oder über eine transdermale Applikationsvornchtung verabreicht werden soll.

6. TRPM8-Aktivierungsmittel zur Verwendung oder Verwendung nach einem der vorhergehenden Ansprüche, wobei das Medikament mit einer anderen Verbindung oder anderen Verbindungen verabreicht werden soll.

## Revendications

1. Agent activateur de canal cationique M8 à potentiel de récepteur transitoire (TRP), sélectionné parmi le groupe consistant en:
(i) iciline (1-(2'-hydroxyphényl)-4-(3"-nitrophényl)-1,2,3,6-tétrahydropyrimidin-2-one); et
(ii) menthol 1R,2S,5R-(5-méthyl-2-[1-méthyléthyl]cyclohexanol);
(iii) iciline comprenant une modification à la position 2' pour inclure un élément sélectionné parmi les groupes consistant en:
(i) alkyloxy en C₁-C₄;
(ii) NO₂;
(iii) =O; et
(iv) NH₂;
(iv) méthoxyiciline;
à utiliser pour induire une analgésie chez un patient souffrant de/ou ressentant une douleur neuropathique chronique.

2. Utilisation d'un agent activateur de canal cationique M8 à potentiel de récepteur transitoire (TRP), dans la fabrication d'un médicament à utiliser dans l'induction d'analgésie chez un patient souffrant de/ou ressentant une douleur neuropathique chronique, où l'agent activateur de canal cationique TRPM8 est sélectionné parmi le groupe consistant en:
(i) iciline (1-(2'-hydroxyphényl)-4-(3"-nitrophényl)-1,2,3,6-tétrahydropyrimidin-2-one); et
(ii) menthol 1R,2S,5R-(5-méthyl-2-[1-méthyléthyl]cyclohexanol);
(iii) iciline comprenant une modification à la position 2' pour inclure un élément sélectionné parmi les groupes consistant en:
(v) alkyloxy en C₁-C₄;
(vi) NO₂;
(vii) =O; et
(viii) NH₂;
(iv) méthoxyiciline.

3. Agent activateur de TRPM8 à utiliser selon la revendication 1 ou à utiliser selon la revendication 2, où l'agent activateur de TRPM8 est du 1R,2S,5R-(5-méthyl-2-[1-méthyléthyl]cyclohexanol).

4. Agent activateur de TRPM8 à utiliser ou utilisation selon l'une quelconque des revendications précédentes, où la douleur neuropathique chronique est sélectionnée parmi les états de douleurs suivantes:
(i) Douleur neuropathique induite par traumatisme.
(ii) Douleur induite par démyélinisation.
(iii) Etats inflammatoires douloureux.
(iv) Douleur de membre fantôme.
(v) Douleur neuropathique associée au cancer.
(vi) Neuropathie induite par chimiothérapie.
(vii) Douleur dorsale.
(viii) Douleur osseuse et douleur osseuse induite par le cancer.

5. Agent activateur de TRPM8 à utiliser ou utilisation selon l'une quelconque des revendications précédentes, où le médicament est destiné à être administré à un site périphérique, localement, par voie intrathécale, comme une épidurale, par voie transdermique ou par un dispositif de délivrance transdermique.

6. Agent activateur de TRPM8 à utiliser ou utilisation selon l'une quelconque des revendications précédentes, où le médicament est destiné à être administré avec un autre composé ou d'autres composés.
